(19) Europäisches Patentamt
European Patent Office
Office européen des brevets

(11) EP 2 098 523 A1

(12) **EUROPEAN PATENT APPLICATION**
published in accordance with Art. 153(4) EPC

(43) Date of publication:
**09.09.2009 Bulletin 2009/37**

(21) Application number: **07830855.8**

(22) Date of filing: **30.10.2007**

(51) Int Cl.:
***C07D 471/04*** *(2006.01)* ***A61K 51/00*** *(2006.01)*

(86) International application number:
**PCT/JP2007/071121**

(87) International publication number:
**WO 2008/059714 (22.05.2008 Gazette 2008/21)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **17.11.2006 JP 2006311203**

(71) Applicant: **NIHON MEDI-PHYSICS CO., LTD.**
**Tokyo 136-0075 (JP)**

(72) Inventors:
- **TANIFUJI, Shigeyuki**
  **Sodegaura-shi**
  **Chiba 299-0266 (JP)**
- **NAKAMURA, Daisaku**
  **Sodegaura-shi**
  **Chiba 299-0266 (JP)**
- **TAKASAKI, Shinya**
  **Sodegaura-shi**
  **Chiba 299-0266 (JP)**
- **OKUMURA, Yuki**
  **Sodegaura-shi**
  **Chiba 299-0266 (JP)**

(74) Representative: **Wilhelms · Kilian & Partner**
**Patentanwälte**
**Eduard-Schmid-Strasse 2**
**81541 München (DE)**

(54) **NOVEL COMPOUNDS WITH AFFINITY FOR AMYLOID**

(57) It is intended to provide a compound effective as an image diagnosis probe targeting amyloid and a diagnostic agent for Alzheimer's disease comprising the compound. Provided is a compound represented by the following formula or a salt thereof:

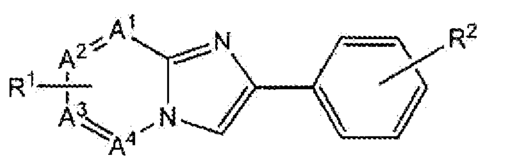

( 1 )

wherein $A^1$, $A^2$, $A^3$ and $A^4$ independently represents a carbon or nitrogen,
$R^1$ is a group selected from the group consisting of hydrogen, hydroxyl group, carboxyl group, sulfuric acid group, amino group, nitro group, cyano group, non-radioactive halogen substituents, alkyl substituents with 1-4 carbon atoms and alkoxy substituents with 1-4 carbon atoms, and$R^3$ is a radioactive halogen substituent,
provided that at least one of $A^1$ $A^2$, $A^3$ and $A^4$ represents a carbon, and $R^1$ binds to a carbon represented by $A^1$, $A^2$, $A^3$ or $A^4$, as well as a diagnostic agent for Alzheimer's disease comprising the compound represented by the above formula or a salt thereof.

EP 2 098 523 A1

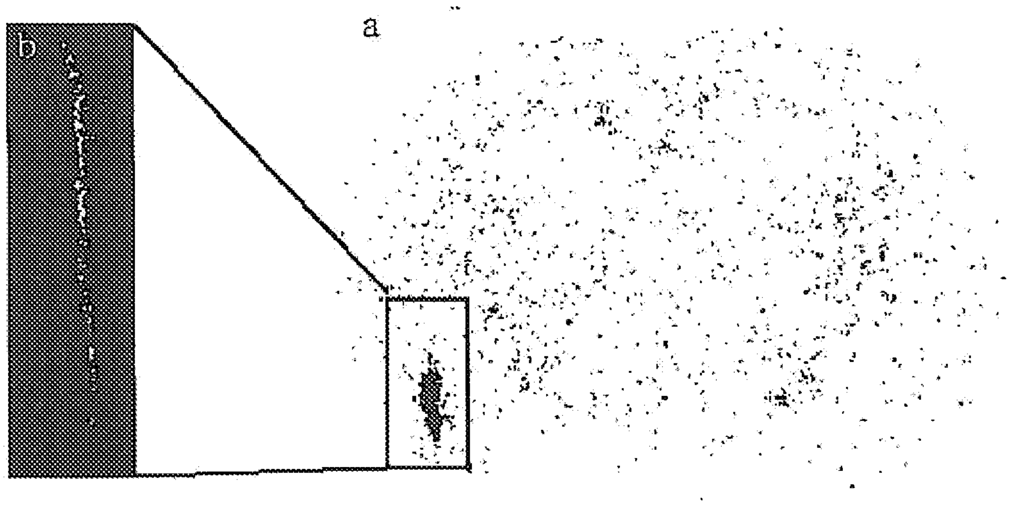


Figure 10

## Description

### TECHNICAL FIELD

The preset invention relates to a compound for use in diagnosis of cerebral degenerative disease. More specifically, the invention relates to a compound useful for amyloid detection at lesion sites in diagnosis of Alzheimer's disease and other diseases with amyloid accumulation.

### BACKGROUND ART

Diseases with the onset of deposition of a fibrous protein called amyloid in various organs or tissues in bodies are generally referred to as amyloidosis. A feature common to amyloidosis is that the fibrous protein called which is enriched with the β-sheet structure is deposited at various organs systemically or at sites topically so that functional abnormalities are triggered in the organs or tissues.
Alzheimer's disease (hereinafter referred to as AD), which is a typical amyloidosis disease, is known as a disease causing dementia. This disease is lethal with progressive deposition of amyloid in brain, and thus is said to be a disease that causes concern in society compared with other amyloidosis diseases. In recent years, the number of AD patients is rapidly increasing in developed countries with aging societies, thereby causing a social problem.
From the pathohistological viewpoint, AD is characterized by three pathological findings in brain, namely development of senile plaques, formation of neurofibrillary tangles, and extensive neuronal loss. The senile plaque has a structure mainly composed of amyloid, and is said to appear at the earliest stage of AD onset and thus is pathologically found in brain about 10 or more years before appearance of clinical symptoms.
AD is diagnosed by carrying out various evaluations of cognitive functions (for example, Hasegawa scale, ADAS-JCog and MMSE) in auxiliary combination with imaging diagnosis such as CT and MRI. However, the method based on such evaluations of cognitive functions is low in diagnostic sensitivity at the early stage of the onset, and is furthermore problematic in that diagnostic results are susceptible to inborn cognitive functions of individuals. At present, it is practically impossible to establish a definite diagnosis of AD while an AD patient is still alive, because the definite diagnosis requires a biopsy of a lesion (Non-Patent Document 1).
Meanwhile, a report tells that amyloid constituting senile plaques is an aggregate of amyloid β protein (hereinafter referred to as Aβ). Also, numerous reports tell that the Aβ aggregate forms a β-sheet structure that causes nerve cell toxicity. Based on these findings, the so-called "Amyloid Cascade Hypothesis" is proposed, which suggests that cerebral deposition of Aβ triggers the downstream phenomena, namely, formation of neurofibrillary tangles and neuronal loss (Non-Patent Document 2).
Based on these facts, attempts have recently been made to detect AD in vivo using a compound having high affinity with amyloid as a marker.
Many of such probes for imaging diagnoses of cerebral amyloid are hydrophobic low-molecular weight compounds that are high in affinity with amyloid and high in cerebral transferability and are labeled with various radioactive species such as $^{11}C$, $^{18}F$ and $^{123}I$. For example, reports tell $^{11}C$ or radioactive halogen labeled forms of compounds including various thioflavin derivatives such as 6-iodo-2-[4'-(N,N-dimethylamino) phenyl]benzothiazole (hereinafter referred to as TZDM) and 6-hydroxy-2-[4'-(N-methylamino)phenyl]benzothiazole (hereinafter referred to as 6-OH-BTA-1) (Patent Document 1, Non-Patent Document 3); stilbene compounds such as (E)-4-methylamino-4'-hydroxystilbene (hereinafter referred to as SB-13) and (E)-4-dimethylamino-4'-iodostilbene (hereinafter referred to as m-I-SB) (Patent Document 2, Non-patent Document 4, Non-Patent Document 5); benzoxazole derivatives such as 6-iodo-2-[4'-(N,N-dimethylamino)phenyl]benzoxazole (hereinafter referred to as IBOX) and 6-[2-(fluoro)ethoxy]-2-[2-(2-dimethylaminothiazol-5-yl)ethenyl]benzoxazole (Non-Patent Document 6, Non-Patent Document 7), DDNP derivatives such as 2-(1-{6-[(2-fluoroethyl)(methyl)amino]-2-naphthyl}ethylidene)malononitrile (hereinafter referred to as FDDDNP) (Patent Document 4, Non-Patent Document 8); and imidazopyridine derivatives such as 6-iodo-2-[4'-(N,N-dimethylamino)phenyl]imidazo[1,2-a]pyridine (hereinafter referred to as IMPY) (Patent Document 3, Non-Patent Document 9). Further, some of these probes for imaging diagnosis have been studied on human imaging and have been reported to show a significant accumulation in AD patient's brain compared with normal persons (Non-patent Document 10, Non-Patent Document 11, Non-Patent Document 12, Non-Patent Document 13).
International Publication No. WO2007/002540 pamphlet discloses a series of compounds with a group having affinity with amyloid, to which a radioisotope labeling site is attached via ethylene glycol or polyethylene glycol (Patent Document 5).
International Publication No. MO2007/063946 pamphlet discloses a series of compounds to which a five-membered aromatic heterocyclic group is attached in order to prevent them from being metabolized in brain.
[Patent Document 1] JP-T-2004-506723
[Patent Document 2] JP-T-2005-504055
[Patent Document 3] JP-T-2005-512945
[Patent Document 4] JP-T-2002-523383
[Patent Document 5] International Publication No, WO2007/002540 pamphlet
[Patent Document 6] International Publication No, WO2007/063946 pamphlet
[Non-Patent Document 1] J. A. Hardy & G. A. Higgins, "Alzheimer's Disease: The Amyloid Cascade Hypothesis.", Science, 1992, 256, p.184-185
[Non-Patent Document 2] G. McKhann et al., "Clinical diagnosis of Alzheimer's disease: Report of the NINCDS-ADRDA Work Group under the auspices of Department of Health and Human Services Task Force on Alzheimer's Disease.", Neurology, 1984, 34, p.939-944
[Non-Patent Document 3] Z.-P. Zhuang et al., "Radioiodinated Styrylbenzenes and Thioflavins as Probes for Amyloid Aggregates.", J. Med. Chem., 2001, 44, p,1905-1914
[Non-Patent Document 4] Masahiro Ono et al., "11C-laheled stilbene derivatives as Aβ-aggregate-specific PET imaging agents for Alzheimer's disease.", NuclearMedicine and Biology, 2003, 30, p.565-571
[Non-Patent Document 5] H. F. Kung et al., "Novel Stilbenes as Probes for amyloid plaques.", J. American Chemical Society, 2001, 123, p.12740-12741
[Non-Patent Document 6] Zhi-Ping Zhuang et al., "IBOX (2- (4'-dimethylaminophenyl)-6- iodobensoxazole): a ligand for imaging amyloid plaques in the brain.", Nuclear Medicine and Biology, 2001, 28, p.887-894
[Non-Patent Document 7] Furumoto Y et al., "[11C]BF-227: A New 11C-Labeled 2-Ethenylbenzoxazole Derivative for Amyloid-β Plaques Imaging.", European Journal of Nuclear Medicine and Molecular Imagine, 2005, 32, Sup.1, P759
[Non-Patent Document 8] Eric D. Agdeppa et al., "2-Dialkylamino-6-Acylmalononitrile Substituted Naphthalenes (DDNP Analogs): Novel Diagnostic and Therapeutic Tools in Alzheimer's Disease.", Molecular Imaging and Biology, 2003, 5, p.404-417
[Non-Patent Document 9] Zhi-Ping Zhuang et al., "Structure-Activity Relationship of Imidazo[1,2-a]pyridines as Ligands for Detecting β-Amnyloid Plaques in the Brain.", J. Med. Chem, 2003, 46, p.237-243
[Non-Patent Document 10] W. E. Klunk et al., "Imaging brain amyloid in Alzheimer's disease with Pittsburgh Compound-B.", Ann. Neurol., 2004, 55, p.306-319
[Non-Patent Document 11] Nicolaas P. L. G. Verhoeff et al., "In-Vivo Imaging of Alzheimer Disease β-Amyloid With [11C] SB-13 PET.", American Journal of Geriatric Psychiatry, 2004, 12, p.584-595
[Non-Patent Document 12] Hiroyuki Arai et al., "[11C]-BF-227 AND PET to visualize Amyloid in Alzheimer's Disease Patients", Alzheimer's & Dementia: The Journal of the Alzheimer's Association, 2006, 2, Sup. 1, S312
[Non-Patent Document 13] Christopher M. Clark et al., "Imaging Amyloid with I123 IMPY SPECT", Alzheimer's & Dementia: The Journal of the Alzheimer's Association, 2006, 2, Sup. 1, S342

## DISCLOSURE OF THE INVENTION

## PROBLEMS TO BE SOLVED BY THE INVENTION

As described above, various compounds are disclosed as probes for imaging diagnosis for amyloid, and researched for clinical application.
Experiments in normal mice show that [$^{125}$I]-labeled TZDM, IBOX and m-I-SB are all transferred into brain 2 minutes after administration. However, these compounds are insufficient in clearance from normal tissues, and tend to accumulate gradually in brain as time passes after administration (JP-T-2005-512945; Zhi-Ping Zhuang et al., Nuclear Medicine and Biology, 2001, 28, p.887-894; H. F. Kung et al., J. Am. Chem. Soc., 2001, 123, p,12740-12741). When the clearance from normal tissues is insufficient, a problem arises in that sufficient contrast cannot be obtained at amyloid accumulation sites.
[$^{11}$C]-labeled SB-13 shows a clearance from normal tissues in experiments in rats, however, it cannot be said that the clearance is sufficiently fast (Masahiro Ono et al., Nuclear Medicine and Biology, 2003, 30, p.565-571).
Meanwhile, it is revealed that compounds having an imidazopyridine skeleton such as IMPY have a property of transferring to brain and accumulating at amyloid after administration, and also have an excellent property of rapid clearance from normal tissues unlike the above-described compounds, as a result of experiments using [$^{125}$I]-labeled compounds. However, IMPY is a compound positive in reverse mutation test. In order to use this compound as a probe for imaging diagnosis, sufficient care must be taken about dosage and administration manner (International Publication No. WOO3/106439 pamphlet).
FDDNP is also reported to be positive in reverse mutation test (International Publication No. WO03/106439 pamphlet).
As described above, various compounds have been disclosed as probes targeting amyloid for imaging diagnosis, but there has been no compound which is confirmed to have a clinically tolerable property.

The present invention has been made under such circumstances, and aims at providing a compound that is effective as a probe targeting amyloid for imaging diagnosis and a diagnostic agent for Alzheimer's disease comprising the compound.

MEANS FOR SOLVING THE PROBLEMS

The inventors have found that a group of compounds usable as probes targeting amyloid for imaging diagnosis can be obtained by labeling a compound having an imidazopyridine-phenyl skeleton directly with a radioactive halogen, and thus have completed the present invention.

Specifically, according to one aspect of the present invention, a compound represented by the following formula (1):

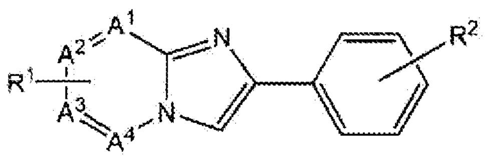

(1)

or a salt thereof, and a diagnostic agent for Alzheimer's disease comprising a compound represented by the above formula (1) or a salt thereof are provided.

In the formula (1), $A^1$, $A^2$, $A^3$ and $A^4$ independently represent a carbon or nitrogen, and it is necessary that at least one of these represents a carbon. Preferably, 3 or more of $A^1$, $A^2$, $A^3$ and $A^4$ represent carbons, and more preferably, all of them represent carbons. In the formula (1), $R^1$ binds to a carbon represented by $A^1$, $A^2$, $A^3$ or $A^4$. A binding site for $R^1$ is preferably a carbon represented by $A^3$, that is, a carbon at 6-position.

In the formula (I) , $R^1$ may be a group selected from the group consisting of hydrogen, hydroxyl group, carboxyl group, sulfuric acid group, amino group, nitro group, cyano group, non-radioactrve halogen substituents, alkyl substituents with 1-4 carbon atoms and alkoxy substituents with 1-4 carbon atoms. $R^1$ is preferably hydroxyl group, alkyl substituents with 1-4 carbon atoms or alkoxy substituents with 1-4 carbon atoms, and more preferably hydroxyl group, methyl substituent or methoxy substituent.

$R^2$ may be a radioactive halogen substituent, preferably a radioactive halogen substituent selected from the group consisting of $^{18}F$, $^{76}Br$, $^{123}I$, $^{124}I$, $^{125}I$ and $^{131}I$ and more preferably a radioactive halogen substituent selected from $^{18}F$, $^{76}Br$, $^{123}I$ and $^{123}I$.

According to the preferable embodiment of the present invention, a compound represented by the following formula (1'):

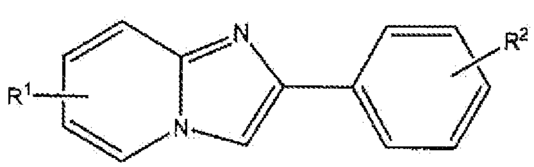

(1')

or a salt thereof and a diagnostic agent for Alzheimer's disease comprising a compound represented by the above formula (1') or a salt thereof are provided.

In the formula (1'), $R^1$ and $R^2$ are respectively the same as in the above formula (1).

According to another aspect of the present invention, a compound represented by the following formula (2):

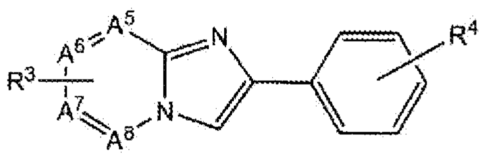

(2)

or a salt thereof, and a diagnostic agent for Alzheimer's disease comprising a compound represented by the above formula (2) or a salt thereof are provided.

In the formula (2), $A^5$, $A^6$, $A^7$ and $A^8$ independently represent a carbon or nitrogen, and it is necessary that at least one of these represents a carbon. Preferably, 3 or more of $A^5$, $A^6$, $A^7$ and $A^8$ represent carbons, and more preferably, all of them represent carbons. In the formula (2), $R^3$ binds to a carbon represented by $A^5$, $A^6$, $A^7$ or $A^9$. A binding site for $R^3$ is preferably a carbon represented by $A^7$ that is, a carbon at 6-position.

In the formula (2), $R^3$ may be a radioactive halogen substituent, preferably a radioactive halogen substituent selected from the group consisting of $^{18}F$, $^{76}Br$, $^{123}I$ $^{124}I$, $^{125}I$ and $^{131}I$, and more preferably a radioactive halogen substituent selected from $^{18}F$, $^{76}Br$, $^{123}I$ and $^{125}I$.

$R^4$ may be a group selected from the group consisting of hydrogen, carboxyl group, sulfuric acid group, nitro group, cyano group, non-radioactive halogen substituents, alkyl substituents with 1-4 carbon atoms and methoxy substituents. $R^4$ is preferably an alkyl substituent with 1-4 carbon atoms or alkoxy substituent with 1-4 carbon atoms, and more preferably methyl substituent or methoxy substituent.

According to the preferable embodiment of the present invention, a compound represented by the following formula (2'):

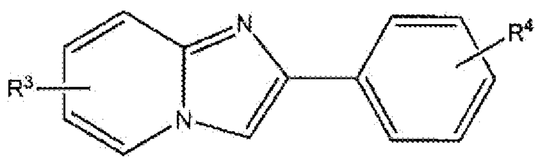

(2')

or a salt thereof, and a diagnostic agent for Alzheimer's disease comprising a compound represented by the above formula (2') or a salt thereof are provided.

In the formula (2'), $R^3$ and $R^4$ are respectively the same as in the above formula (2).

According to still another aspect of the present invention, a compound represented by the following formula (3):

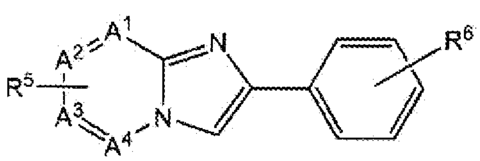

(3)

or a salt thereof is provided.

In the formula (3), $A^1$ $A^2$, $A^3$ and $A^4$ independently represent a carbon or and it is necessary that at least one of these represents a carbon. Preferably, 3 or more of $A^1$, $A^2$, $A^3$ and $A^4$ represent carbons, and more preferably, all of them represent carbons. In the formula (3), $R^1$ binds to a carbon represented by $A^1$, $A^2$ $A^3$ or $A^4$ A binding site for $R^1$ is represented by $A^1$, $A^2$, $A^3$ or $A^4$. A binding site for $R^1$ is preferably a carbon represented by $A^3$, that is, a carbon at 6-position.

In the formula (3), $R^5$ may be a group selected from the group consisting of hydrogen, hydroxyl group, carboxyl group, sulfuric acid group, amino group, nitro group, cyano group, non-radioactive halogen substituents, alkyl substituents with 1-4 carbon atoms and alkoxy substituents with 1-4 carbon atoms. $R^5$ is preferably hydroxyl group, alkyl substituents with 1-4 carbon atoms or alkoxy substituents with 1-4 carbon atoms, and more preferably hydroxyl group, methyl substituent or methoxy substituent.

$R^6$ is a group selected from the group consisting of non-radioactive halogen substituents, nitro group, trialkylstannyl substituents having alkyl chains with 1 to 4 carbon atoms, triphenylstannyl group and trialkylammonium groups having alkyl chains with 1 to 4 carbon atoms.

As a non-radioactive halogen substituent, a halogen capable of being a target oaf nucleophilic substitution reactions using a radioactive fluorine or a radioactive iodine can be used, and preferably chlorine, iodine or bromine can be used.

According to the preferable embodiment of the present invention, a compound represented by the following formula (3'):

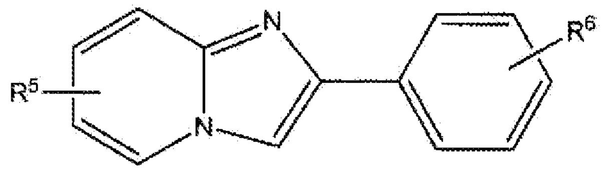

(3')

or a salt thereof is provided.

In the formula (3'), $R^5$ and $R^6$ are respectively the same as in the above formula (3).

According to still another aspect of the present invention, a compound represented by the following formula (4):

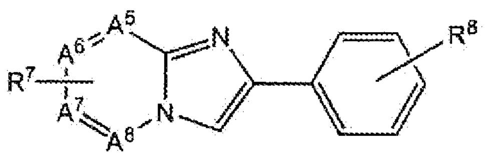

(4)

or a salt thereof is provided.

In the formula (4), $A^5$, $A^6$, $A^7$ and $A^8$ independently represent a carbon or nitrogen, and it is necessary that at least one of these represents a carbon. Preferably, 3 or more of $A^5$, $A^6$, $A^7$ and $A^8$ represent carbons, and more preferably, all of them represent carbons. In the formula (4), $R^7$ binds to a carbon represented by $A^5$, $A^6$, $A^7$ or $A^9$. A blinding site for $R^8$ is preferably a carbon represented by Air that is, a carbon at 6-position.

In the formula (4), $R^7$ is a group selected from the group consisting of non-radioactive halogen substituents, nitro group, trialkylstannyl substituents having alkyl chains with 1 to 4 carbon atoms, triphenylstannyl group and trialkylammonium groups having alkyl chains with 1 to 4 carbon atoms.

As a non-radioactive halogen substituent, a halogen capable of being a target of nucleophilic substitution reactions using a radioactive fluorine or a radioactive iodine can be used, and preferably chlorine, iodine or bromine can be used.

$R^8$ may be a group selected from the group consisting of hydrogen, carboxyl groups sulfuric acid group, nitro group, cyano group, non-radioactive halogen substituents, alkyl substituents with 1-4 carbon atoms and methoxy substituent , $R^8$ is preferably an alkyl substituent with 1-4 carbon atoms or alkoxy substituent with 1-4 carbon atoms, and more preferably methyl substituent or methoxy substituent.

According to a preferable embodiment of the present invention, a compound represented by the following formula (4'):

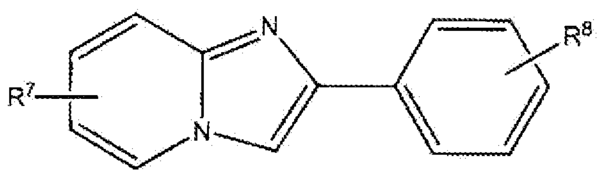

(4')

or a salt thereof is provided.

In the formula (4'), $R^7$ and $R^8$ are respectively the same as in the above formula (4).

EFFECTS OF THE INVENTION

The present invention provides a. novel compound that has affinity with amyloid, and thus can provide a diagnostic agent for Alzheimer's disease comprising the compound.

BEST MODE FOR CARRYING OUT THE INVETION

(A method for synthesis of a precursor compound for a radioactive halogen-labeled compound)
Hereinafter, a method for synthesis of a precursor compound for a radioactive halogen-labeled compound according to an embodiment of the present invention will be described, taking the case of 2-(4'-tributylstannylphenyl)-6-methoxyimidazo[1,2-a]pyridine as an example.
First, 2-bromo-3-hydroxypyridine is allowed to react with methyl iodide in the presence of sodium methoxide to synthesize 2-bromo-3-methoxypyridine. Then, it is subjected to nitration with a mixed acid of conc. sulfuric acid and conc. nitric acid to convert it into 2-bromo-3-methoxy-6-nitropyridine, and then subjected to reductive elimination of the bromo group and reduction of the nitro group using palladium-carbon to synthesize 2-amino-5-methoxypyrldine (Fig. 1, Steps 1-3). In the series of reactions, the reaction conditions can be set in accordance with ordinary methods, for example, the method described in a literature, Joseph G. Lombardino, Journal of Medicinal Chemistry, 1981, 24, p. 39-42.
The obtained 2-amino-5-methoxypyridine is allowed to react with 2-bromo-4'-bromoacetophenone to brominate 4'-position, thereby synthesizing 2-(4'-bromophenyl)-6-methoxyimidazo[1,2-a]pyridine (Fig. 1, Step 4), This step can be conducted according to the following procedure.
First, 2-bromo-4'-bromoacetophenone and 2-amino-5-methoxypyridine are dissolved in an inactive solvent such as acetonitrile, and are allowed to react with each other at a reflux temperature for 2 to 6 hours to produce 2-(4'-bromophenyl-6-methoxyimidazo[1,2-a]pyridine hydrobromide salt has white precipitates. The inactive solvent used in this instance may be acetonitrile or another solvent that is usually employed in a similar reaction, for example, methanol and acetone. The reaction temperature may be a temperature allowing refluxing, for example, 90˚C when the solvent is acetonitrile. The amount of the solvent to be used may be an amount sufficient to effect the reaction, however, it should be noted that if the solvent ifs too much, tit will become difficult to obtain precipitates of reaction products. For example, when 2-bromo-4'-bromoacetophenone in an amount corresponding to 10 mmol is used for the reaction, the amount of a solvent two be used can be about 40 to 50 mL.
Next, the reaction solution is filtered to recover the precipitates. The white precipitates are suspended in a mixed solution of methanol/water (1:1). Then, an aqueous saturated solutions of sodium hydrogencarbonate is added thereto in a very excessive amount relative to the suspended precipitates to release 2-(4'-bromophenyl)-6-methoxyimidazo[1,2-a]pyridine as precipitates. The newly generated precipitates are filtered to remover 2-(4'-bromophenyl)-6-methoxyimidazo[1,2-a]pyridine as the target compound in this step as crystals. The amount of the mixed solution of water/methanol is not specifically limited as long as it is sufficient to effect the reaction. However, it should be noted that if the amount of the mixed solution is too much, precipitation of crystals will be hindered. For example, when 2-bromo-4'-bromoacetophenone in an amount corresponding to 10 mmol is used, the mixed solution of water/methanol may be used in an amount of about 40 to 100 mL. The amount of sodium hydrogencarbonate is not specifically limited as long as it is very excessive relative to the above-described precipitates as reaction substrates. For example, when the reaction is effected under the above-described conditions, the amount of an aqueous saturated solution of sodium hydrogencarbonate to be added to the reaction solution can be about 25mL.
The obtained 2-(4'-bromophenyl)-6-methoxyimidazo[1,2-a]pyridine is dissolved in dioxane, and triethylamine is added to the solution, followed by addition of bis(tributyltin) and a catalytic amount of tetrakis-triphenylphosphine palladium. This reaction solution is heated at about 90˚C to effect reaction, and then a solvent is distilled off and a chromatographic purification is performed to obtain 2-(4'-tributylstannylphenyl)-6-methoxyimidazo[1,2-a]pyridine as the target compound (Fig. 1, Step 5). The amount of bis (tributyltin) to be used in this instance may be an amount satisfying a condition where it is excessive relative to the reaction substrate, specifically, it is about 1.5 times in molar ratio relative to the reaction substrate 2-(4'-bromophenyl)-6-methoxyimidazo[1,2-a]pyridine.
When a compound with a substituent at the 4'-postion being a trialkylstannyl substituent other than tributylstannyl substituent is obtained, various bis(trialkyltin)s that fit purposes can be used instead of bis (tributyltin) in Step 5. For example, when a compound having a trimethylstannyl substituent as a substituent at the 4'-position is synthesized, a reaction similar to the above can be performed in Step 4 using bis (trimethyltin).
A compound with a substituent attached to an imidazo pyridine ring being a hydroxyl substituent can be obtained, for example, by reacting 2-(4'-bromophenyl)-6-methoxyimidazo[1,2-a]pyridine obtained above in Step 4 with boron tribromide to perform demethylation, and then conducting the reaction of the above Step 5. For a compound with a substituent attached to an imidazo pyridine ring being a methyl substituent or ethoxy substituent, 2-amino-5-methylpyridine and 2-amino-ethoxypyridine can respectively be used in the above Step 4 instead of 2-amino-5-methoxypyridine.
A compound with an imidazo pyridine ring in which the binding site for the functional group is a carbon atom

other than the carbon at 6-position can be obtained by using a compound with a pyridine ring to which an alkoxy substituent or the like is bonded at a different, site instead of 2-amino-5-methoxypyridine in Step 4. For example, when a binding site for the functional group is the carbon at 8-position in the imidazo pyridine ring, 2-amino-3-methoxypyridine may be used instead of 2-amino-5-methoxypyridine in Step 4 in the above example.

Further, a labeling precursor compound with a radioactive halogen labeled site being an imidazo pyridine ring, for example, 6-tributylstannyl-2-(4'-methoxyphenyl)imidazo[1,2-a] pyridine can be synthesized by the following procedure.

First, 4'-hydroxyacetophenone is allowed to react with cupric bromide to prepare 2-bromo-4'-hydroxyacetophenone in accordance with ordinary methods, for example, the method described in a literature, King, L. Carroll and Ostrum, G. Kenneth, Journal of Organic Chemistry, 1964, 29(12), p.3459-3461 (Fig. 3, Step 1). This is allowed, to react with 2-amino-5-iodopyridine in an inactive solvent such as acetonitrile, and the obtained precipitation is purified to obtain 2-(4'-hydroxyphenyl)-6-iodoimidazo[1,2-a]pyridine (Fig. 3, Step 2). The condition in this step may be the same condition as described above in Step 4 of Fig. 1.

Then, the obtained 2-(4'-hydroxyphenyl)-6-iodoimidazo[1,2-a]pyridine is dissolved in a mixed solution of methanol and dioxane, and trimethylsilyl diazomethane is added thereto to allow reaction. The solvent is distilled off, and the residue is purified to synthesize 6-iodo-2-(4'-methoxyphenyl)imidazo[1,2-a]pyridine (Fig. 3, Step 3). In this step, the amount of trimethylsilyl diazomethane may be not less than the amount of 2-(4'-hydroxyphenyl)-6-iodoimidazo [1,2-a] pyridine.

The obtained 6-iodo-2-(4'-methoxyphenyl)imidazo[1,2-a]pyridine is dissolved in dioxane, and triethylamine is added the solution, followed by addition of bis (tributyltin) and a catalytic amount of tetrakis-triphenylphosphine palladium to obtain 6-tributylstannyl-2-(4'-methoxyphenyl)imidazo[1,2-a]pyridine as the target compound (Fig. 4, Step 1). The condition in this step may be the same condition as described above in Step 5 of Fig. 1.

(A method for synthesis of a radioactive halogen-labeled compound)

Hereinafter, a method for production of a radioactive halogen-labeled compound according to another aspect of the present invention will be described, taking the case of radioactive iodine-labeled compounds as an example.

The synthesis of radioactive iodine-labeled compounds can be performed by dissolving the labeling precursor compound prepared as above procedure in an inert organic solvent, adding thereto a [$^{123}$I] sodium iodide solution or the like obtained by known methods, and adding thereto an acid and an oxidizing agent to effect reaction. As an inert organic solvent dissolving the labeling precursor compound, various solvents having no reactivity with the labeling precursor, [$^{123}$I] sodium iodide and the like can be used, and preferably methanol can be used.

As the acid, may be used various ones, and preferably hydrochloric acid.
The oxidizing agent is not particularly limited as long as it can effect the oxidation of iodine in the reaction solution, and is preferably hydrogen peroxide or peracetic acid. The amount of the oxidizing agent to be added may be an amount sufficient to oxidize iodine in the reaction solution.

A compound labeled with a radioactive halogen ether than iodine can be synthesized by labeling a labeling precursor that fits a purpose of synthesis with a radioactive halogen that fits the purpose. For example, in order to synthesize 2-(4'-[$^{18}$F] fluorophenyl)-6-methoxyimidazo[1,2-a]pyridine, the labeling precursor 2-(4'-nitrophenyl)-6-methoxyimidazo[1,2-a]pyridine can be reacted with [$^{18}$F] fluoride ion in the presence of a phase transfer catalyst and potassium carbonate.

(Methods for prepaying and using a diagnostic agent in accordance with the present invention)

The diagnostic agent according to the present invention can be prepared as a solution which comprises the present radioactive halogen-labeled compound blended in Mater, a physiological saline solution or a Ringer's solution optionally adjusted two an appropriate pH, like other commonly-known radioactive diagnostic agents. In this instance, concentration of the present compound should be adjusted to not more than the concentration at which stability of the present compound is ensured. Dosage of the present compound is not specifically limited as long as it is sufficient to obtain an image of distribution of an administered agent. For example, in case of iodine-123-labeled compounds and fluorine-18-labeled compounds, about 50 to 600 MBq per adult body of 60 kg weight can be administered intravenously or locally. Distribution of administered agents can be imaged by known methods. For example, iodine-123-labeled compounds can be imaged by a SPECT apparatus while fluorine-18-labeled compounds can be imaged by a PET apparatus,

Hereinafter, the present invention is described bellow in more detail by way of Examples, Comparative Examples and Reference Examples. However, these Examples never the scope of the present invention.

Example 1: synthesis of 2-(4'-tributylstannylphenyl)-6-methoxyimidazo[1,2-a]pyridine

100.0 g (corresponding to 0.575 mol) of 2-bromo-3-hydroxypyridine was dissolved in 310 mL of and 575 mL

(corresponding to 0.575 mol) of a 1 mol/L, sodium methoxide-methanol solution was added thereto. Then, the reaction solution was heated to 90˚C to distill off methanol. After the reaction solution was cooled down to 10˚C or lower, 93.9 g (corresponding to 0.662 mol) of methyl iodide was added, and then stirred at room temperature for 20.5 hours. After the completion of the reaction, the reaction solution was poured into ice water and extracted twice with chloroform. The combined chloroform layer was washed with a 1 mol/L sodium hydroxide solution, washed twice with a saturated sodium chloride solution, and dried over anhydrous sodium sulfate. After the solvent was distilled off under reduced pressure, 65.4 g (corresponding to 0.348 mol) of 2-bromo-3-methoxypyridine was obtained (Fig. 1, Step 1).

262 mL of conc. sulfuric acid was cooled down to -2˚C, and 262 mL of 90 % nitric acid was carefully added thereto. Subsequently, 65.3 g (corresponding to 0.347 mmol) of 2-bromo-3-methoxypyridine was carefully added thereto. After the reaction mixture was stirred in an ice bath for 10 minutes, the mixture was stirred at room temperature for 30 minutes, and then was heated to 55 ˚C and further stirred for 1.5 hours. After the reaction solution was cooled, the reaction solution was poured little by little into crushed ice to generate precipitates. The precipitates were filtered and washed with water, and then dried over phosphorous pentoxide under reduced pressure, to obtain 55.7 g (corresponding to 0.239 mmol) of 2-bromo-3-methoxy-6-nitropyridine (Fig. 1, step 2).

55.6 g (corresponding to 0.239 mol) of was dissolved in 1700 mL of ethanol, and 37.3 g (50%. wet) of 10 % palladium-carbon was added thereto under argon stream. To the mixture, 283 mL of hydrazine monohydrate was then added dropwise. After the reaction mixture was heated under reflux for 70 minutes, the reaction solution was cooled down to room temperature. Then, after palladium-carbon was filtered off, the residue was washed with ethanol, and the washings were combined with the filtrate. The combined solution was concentrated under reduced pressure. Then, 1300 mL of water and 130 mL of conc. aqueous ammonia were added to the concentrate, and the resulting mixture was extracted eight times with chloroform. The combined chloroform layer was dried over anhydrous sodium sulfate and concentrated under reduced pressure. The resulting crude product was distilled under reduced pressure to obtain 26.2 g (corresponding to 0.211 mol) of 2-amino-5-methoxypyridine (Fig. 1, Step 3).

844 mg (corresponding to 3.0 mmol) of 2-bromo-4'-bromoacetophenone and 378 mg (corresponding to 3.0 mmol) of 2-amino-5-methoxypyridine were dissolved in 25 mL of acetonitrile. The resulting solution was heated under reflux in an oil bath at 105˚C for 3.5 hours. After the completion of the reaction, the reaction solution was cooled down to room temperature, and precipitates were filtered and recovered. The filtered precipitates were washed with acetonitrile and dried under reduced pressure to obtain crude crystals. The resulting crude crystals were suspended in a mixed solution of 7 mL of water and 7 mL of methanol. Then, about 7 mL of a saturated sodium hydrogencarbonate solution was added thereto, and the mixture was sonicated for 5 minutes using an ultrasonic washing machine. Precipitates were filtered, sufficiently Mashed with Mater, and dried under reduced pressure, to obtain 640 mg (corresponding to 2.11 mmol) of 2-(4'-bromophenyl)-6-methoxyimidazo[1,2-a]pyridine (Fig. 1, step 4).

463 mg (corresponding to 1.5 mmol) of 2-(4'-bromophenyl)-6-methoxyimidazo[1,2-a]pyridine was dissolved in 25 mL of dioxane, and 2 mL of triethylamine was added thereto. Then, 1.15 mL (corresponding to 2.25 of bis(tributyltin) and 19 mg (a catalytic amount) of tetrakis-triphenylphosphine palladium were added thereto. After the reaction mixture was stirred at 90˚C for 15 hours, the solvent was distilled off under reduced pressure. The residue was purified by flash silica gel column chromatography (elution solvent: hexane/ethyl acetate = 5/1→4/1). Further, the resulting crude product was purified by recycle preparative HPLC (HPLC apparatus: LC-908 (under trade name: manufactured by Japan Analytical Industry Co., Ltd.); column: two JAIGEL 2H (under trade name; manufactured by Japan Analytical Industry Co., Ltd.) connected together; mobile phase: chloroform), to obtain 419 mg (corresponding to 0.82 mmol) of 2-(4'-tributylstannylphenyl)-6-methoxyimidazo[1,2-a]pyridine (Fig. 1, Step 5).

The NMR measurement results of the resulting 2-(4'-tributylstannylphenyl)-6-methoxyimidazo [1,2-a]pyridine (internal standard: tetramethylsilane) are shown below.

MMR apparatus employed: JNN-ECP-500 (manufactured by Japan Electron Optics Laboratory Co., Ltd. (JEOL)) $^{1}$H-NMR (solvent: chloroform-dl, resonance frequency: 500MHz): 57.89-7.84 (m, 2H), 7.81 (s, 1H), 7.66-7.63 (m, 1H), 7.57-7.46 (m, 3H), 6.96 (dd, J = 9.7,2.4 Hz, 1H), 3.83 (s, 3H), 1.64-1.47 (m, 6E), 1.34 (sextet, J = 7.3 Hz, 6H), 1.15-1.100 (m, 6H), 0.89 (t, J = 7.3 Hz, 9H).

$^{13}$C-NMR (solvent: chloroform-dl, resonance frequency: 125 MHz): δ 1.49.29, 146.02, 142.90, 141.71, 136.84, 133.52, 125.23, 119.66, 117.73, 109.16, 107.47, 56.20, 29.12, 27.38, 13.69, 9.62.

Example 2: Synthesis of [$^{123}$I]-2-(4'-iodophenyl)-6-methoxyimidazo[1,2-a]pyridine

To 75 μL of a solution of 2-(4'-tributylstannylphenyl)-6-methoxyimidazo[1,2-a]pyridine in methanol (concentration: 1 mg/mL), 100 μL of 1. mol/L hydrochloric acid, 10 μL of 1 mmol/L sodium iodide, [$^{123}$I] sodium iodide solution of 160 MBq (80 μL in volume) and 20 μL of 10% (w/v) hydrogen peroxide were added. After the mixed solution was left to stand at 50˚C for 10 minutes, the solution was subjected to HPLC under the following conditions, to obtain a fraction of [$^{123}$I]-2-(4'-iodophenyl)-6-methoxyimidazo[1,2-a]pyridine.

HPLC conditions:

Column: Phenomenex Luna C18 (trade name; manufactured by Phenomenex Co.; size: 4.6 x 150 mm)

Mobile phase: 0.1 % trifluoroacetic acid/acetonitrile = 20/80 to 0/100 (17 minutes, linear gradient)
Flow rate: 1.0 mL/min.
Detector: Ultraviolet visible absorptiometer (Detection wavelength: 282 nm) and radioactivity counter (manufactured by raytest: type STEFFI)

10 ml of water was added to the faction. The resulting solution was passed through a reversed phase column (trade name:
Sep-Pak (registered trademark) Light C8 Cartridges manufactured by Waters: the packed amount of the packing agent: 130 mg) so that the column adsorbs and collects [$^{123}$I]-2-(4'-iodophenyl)-6-methoxyimidazo[1,2-a]pyridine. The column was rinsed with 1 mL of water, and then 1 mL of diethyl ether was passed therethrough to elute [$^{123}$I]-2-(4'-iodophenyl)-6-methoxyimidazo[1,2-a]pyridine. The amount of radioactivity of the obtained compound was 27 MBq (at the end of production). Further, the TALC analysis was conducted under the following conditions, and as a result, the radiochemical purity off the compound was 97%.
TLC analysis conditions:

TLC plate: RP-18F254 (trade name; manufactured by Merck & Co., Inc.)
Mobile phase: chioroform/methanol/triethylamine = 100/1/2
Detector: Bio-imaging Analyzer, BAS-2500 (type: BAS-2S00 manufactured by FUJIEILM Corporation)

Example 3: synthesis of [$^{123}$I]-2-(4'-iodohenyl)-6-methoxyimidazo[1,2-a]pyridine
To 75 μL of a solution of 2-(4'-tributylstannylphenyl)-6-methoxyimidazo[1.2-apyridine in methanol (concentration: 1 mg/mL), 100 μL of 1 mol/L hydrochloric acid 10 μL of 1 mmol/L sodium iodine, [$^{125}$I]H sodium iodide solution of 32 MBq (20 μL in volume) and 20 μL of 10% (w/v) hydrogen peroxide were added. After the mixed solution was left to stand at 50°C for 10 minutes, the solution was subjected to HPLC under the same conditions as in Example 2, to obtain a fraction of [$^{125}$I]-2-(4'-iodophenyl)-6-methoxyimidazo[1,2-a]pyridine.
10 ml of water was added to the fraction. The resulting Solution was passed through a reversed phase column (trade name: Sep-Pak (registered trademark) Light C8 Cartridges manufactured by Waters: the packed amount of the packing agent: 130 mg) so that the column adsorbs and collects [$^{125}$I]-2-(4'-iodophenyl)-6-methoxyimidazo[1,2-a]pyridine. The column was rinsed, with 1 mL of water, and then 1 mL of methanol was passed therethrough to elute [$^{125}$I]-2-(4'-iodophenyl)-6-methoxyimidazo[1,2-a]pyridine. The amount of radioactivity of the obtained compound was 15 MBq (at the end of production). Further, the TLC analysis was conducted under the same conditions as in Example 2, and as a result, the radiochemical purity off the compound was 92%.
Example 4: Synthesis of 2-(4'-iodophenyl)-6-methoxyimidazo[1,2-a]pyridine (non-radioactive iodinated form)
100 mg (corresponding to 0.20 mmol) of 2-(4'-tributylstannylphenyl)-6-methoxyimidazo[1,2-a]pyridine obtained in Example 1 was dissolved, in 2.0 mL of dichloromethane, and 37 mg (corresponding to 0.29 mmol) of iodine was added thereto. After the reaction mixture was stirred at 0°C for 30 minutes, a saturated sodium hydrogencarbonate aqueous solution and a saturated sodium thiosulfate aqueous solution were added and extracted three times with dichloromethane. The combined dichloromethane layer was dried over anhydrous sodium sulfate, and concentrated under reduced pressure. The resulting crude product was purified by silica gel column chromatography (elution solvent: hexane/ethyl acetate = 2/1) to obtain 44.5 mg (corresponding to 0.13 mmol) of 2-(4'-iodophenyl)-6-methoxyimidazo[1,2-a]pyridine (Fig. 2, Step 1).
The NMR measurement results of the resulting 2-(4'-iodophenyl)-6-methoxyimidazo[1,2-a]pyridine (internal standard: tetramethylsilane) are shown below.
NMR apparatus employed: JNM-ECP-500 (manufactured by Japan Electron Optics Laboratory Co., Ltd. (JEOL)) $^{1}$H-NMR (solvent: chloroform-dl, resonance frequency: 500MHz): δ 7.75-7.71 (m, 3H), 7.64-7.62 (m, 2H), 7.59 (d, J = 1.8 Hz, 1H), 7.49 (d, J = 10.1 Hz, 1H), 6.96 (dd, J = 10.1, 1.8 Hz, 1H), 3.81 (s, 3H).
$^{13}$C-NMR (solvent: chloroform-dl, resonance frequency: 125 MHz): 5149.80, 144.94, 143.27, 138.12, 133.91, 127.88, 120.56, 118.05, 109.72, 107.79, 93.45, 56.57.
Example 5: Synthesis of 6-iodo-2-(4'-methoxyphenyl)imidazo[1,2-a]pyridine (non-radioactive iodinated form)
50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was heated under reflux. After 5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.25 g (corresponding to 33.7 mmol) of 2-bromo-4'-hydroxyacetophenone (Fig. 3, Step 1).

441 mg (corresponding to 2.0 mmol) of 2-bromo-4'-hydroxyacetophenone and 449 mg (corresponding to 2.0 of 2 -amino-5-iodc)pyridine were dissolved in 15 mL of acetonitrile. The resulting solution was heated under reflux in an oil bath at for 5 hours. After the completion of the reaction, the reaction solution was cooled down to room temperature, and precipitates were filtered and recovered. The precipitates were washed with acetonitrile and dried under reduced pressure. The resulting crude crystals were suspended ins mixed solution of 10 mL of water and 10 mL of methanol. Then, about 10 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 0.53 g (corresponding to 1.56 mmol) of 2-(4'-hydtoxyphenyl)-6-iodoimidazo[1,2-a]pyridine (Fig. 3, Step 2).

0.40 g (corresponding to 1.2 mmol) of 2-(4'-hydroxyphenyl)-6-iodoimidazo[1,2-a]pyridine was dissolved in a mixed solution of 30 mL of methanol and 50 mL of dioxane, and 1.2 mL (corresponding to 2.4 mmol) of trimethylsilyl diazomethane (2M hexane solution) was added thereto. The reaction mixture was stirred at room temperature for 24.5 hours, and the solvent was removed under reduced pressure. The residue was recrystallized from ethyl acetate to obtain 223 mg (corresponding to 0.64 mmol) of 6-iodo-2-(4'-methoxyphenyl)imidazo[1,2-a]pyridine (Fig. 3, Step3).

The NMR measurement results of the resulting 6-iodo-2-(4'-methoxyphenyl)imidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^{1}$H-NMR (solvent: chloroform-dl, resonance frequency: 500MHz): $\delta$ 8.37-8.34 (m, 1H) 7.88-7.84 (m, 2H), 7.72 (s, 1H), 7.40 (d, J = 9.4 Hz, 1H), 7.31 (dd, J = 9.4, 1.6 Hz, 1H), 6.99-6.95 (m, 2H), 3.86 (s, 3H).

$^{13}$C-NMR (solvent: chloroform-dl, resonance frequency: 125 MHz): $\delta$ 159.86, 146.32, 144.18, 132.32, 130.28, 127.41, 125.90, 118.32, 114.22, 106.88, 74.76, 55.33.

Example 6: synthesis of 6-tributylstannyl-2-(4'-methoxyphenyl)imidazo[1,2-a]pyridine

87.6 mg (corresponding to 0.25 mmol) of 6-iodo-2-(4'-methoxyphenyl)imidazo[1,2-a]pyridine obtained in Example 5 was dissolved in 10 mL of dioxane, and 2 mL of triethylamine was added whereto. Then, 190 $\mu$L (corresponding to 0.375 mmol) of bis(tributyltin) and 20 mg (a catalytic amount) of tetrakis-triphenylphosphine palladium were added thereto. After the reaction mixture was stirred at 90°C for 21.5 hours, the solvent was distilled off under reduced pressure. The residue was purified by flash silica gel column chromatography (elution solvent: hexane/ethyl acetate = 3/1). Further, the resulting crude product was purified by recycle preparative HPLC (HPLC apparatus: LC-908 (under trade name: manufactured by Japan Analytical Industry Co., Ltd.); column: two JAIGEL 2H (under trade name; manufactured by Japan Analytical Industry Co., Ltd.) connected together; mobile phase: chloroform), to obtain 53 mg (corresponding to 0.10 mmol) of 6-tributylstannyl-2-(4'-methoxyphenyl)imidazo[1,2-a]pyridine (Fig. 4, Step 1).

The NMR measurement results of the resulting 6-tributylstannyl-2-(4'-methoxyphenyl)imidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^{1}$H-NMR (solvent: chloroform-dl, resonance frequency: 500 MHz): $\delta$ 8.01-7.93 (m, 1H), 7.92-7.87 (m, 2H), 7.74 (s, 1H), 7.60-7.56 (m, 1H), 7.18-7.09 (m, 1H), 6.99-6.94 (m, 2H), 3.84 (s, 3H), 1.66-1.46 (m, 6H), 1.35 (sextet, J = 7.3 Hz, 6M), 1.19-1.02 (m, 6H), 0.91 (t, J = 7.3 Hz, 9H).

$^{13}$C-NMR (solvent: chloroform-dl, resonance frequency: 125 MHz): $\delta$ 159.45, 145.56, 145.01, 131.00, 129.95, 127.22, 126.70, 121.69, 116.80, 114.06, 106.24, 55.23, 28.94, 27.24, 13.59, 9.74.

Example 7: Synthesis of [$^{123}$I]-6-iodo-2-(4'-methoxyphenyl)imidazo[1,2-a]pyridine

To 50 $\mu$L of a solution of 6-tributylstannyl-2-(4'-methoxyphenyl)imidazo[1,2-a]pyridine in methanol (concentration: 1 mg/mL), 50 $\mu$L of 1 mol/L hydrochloric acid, 10 $\mu$L of 1 mmol/L sodium iodide, [$^{123}$I] sodium iodide solution of 318 MBq (50 $\mu$L in volume) and 10 $\mu$L of 10% (w/v) hydrogen peroxide were added. After the mixed solution was left to stand at 50°C for 10 minutes, the solution was subjected to HPLC under the same conditions as in Example 2, to obtain a fraction of [$^{123}$I]-6-iodo-2-(4'-methoxyphenyl)imidazo[1,2-a]pyridine.

10 ml of water was added to the fraction. The resulting solution was passed through, a reversed phase column (trade name: Sep-Pak (registered trademark) Light C18 Cartridges manufactured by waters: the packed amount of the packing agent: 130 mg) so that the column adsorbs and collects [$^{133}$I]-6-iodo-2-(4'-methoxyphenyl)imidazo[1,2-a]pyridine. The column was rinsed with 1 mL of water, and then 1 mL of diethyl ether was passed therethrough to elute [$^{123}$I]-6-iodo-2-(4'-methoxyphenyl)imidazo[1,2-a]pyridine. The amount of radioactivity of the obtained compound was 86 MBq (at the end of production). Further, the TLC analysis was conducted under the same conditions as in Example 2, and as a result, the radiochemical purity of the compound was 98%.

Example 8: Synthesis of 2-(4'-fluorophenyl)-6-iodoimidazo[1,2-a]pyridine (non-radioactive iodinated form)

40 mL of ethyl acetate was added to 3.70 g (corresponding to 16. 6 mmol) of cupric bromide to obtain a suspension, to which a solution of 1.0 mL (corresponding to 8.27 mmol) of 4'-fluoroacetophenone was added. Then, the resulting mixture was heated under reflux. After 3 hours, the reaction mixture was cooled down to room temperature and filtered. The resulting filtrate was concentrated under reduced pressure. The residue was dissolved in ethyl acetate and concentrated. The resulting crude product was purified by silica gel column chromatography (elution solvent: hexane/ethyl acetate = 10/1), to obtain 1.82 g (corresponding to 8.39 mmol) of 2-bromo-4'-fluoroacetophenone (Fig. 5, Step 1).

1.82 g (corresponding to 8.39 mmol) of 2-bromo-4'-fluoroacetophenone and 1.29 g (corresponding to 5.87 mmol) of 2-amino-5-iodopyridine were dissolved in 40 mL of acetonitrile. The resulting solution was heated under reflux in an oil bath at 110°C for 1.5 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 20 mL of water and 10 mL of methanol. Then, about 30 mL of a saturated sodium hydrogencarbonate solution was added thereto, and the mixture was sonicated for 5 minutes using an ultrasonic washing machine. Precipitates were filtered, and recovered from the resulting mixture, sufficiently washed with water, and dried under reduced pressure, to obtain 1.28 g (corresponding to 3.79 mmol) of 2-(4'-fluorophenyl)-6-iodoimidazo[1,2-a]pyridine (Fig. 5, Step 2).

The NMR measurement results of the resulting 2-(4'-fluorophenyl)-6-iodoimidazo[1,2-a]pyridine (internal standard: tetramethyl silane) are shown below.

NMR apparatus employed: JNM-ECP-500 (manufactured by Japan Electron Optics Laboratory Co., Ltd. (JEOL)) $^1$H-NMR (solvent: dimethylformamide-d7, resonance frequency: 500 MHz): δ 9.06 (s, 1H), 8.44 is, 1H), 8.01-7.98 (m, 2H), 7.72 (d, J = 9.6 Hz, 1H), 7.57 (d, J = 9.6 Hz, 1H), 7.38-7.34 (m, 2H).

Example 9: Synthesis of 6-tributylstannyl-2-(4'-fluorophenyl)imidazo[1,2-a]pyridine

338 mg (corresponding to 1.00 mmol) of 2-(4'-fluorophenyl)-6-iodoimidazo[1,2-a]pyridine obtained in Example 8 was dissolved in 4.0 mL of dioxane, and 2 mL of triethylamine was added thereto. Then, 0.76 mL (corresponding to 1.5 mmol) of bis(tributyltin) and 76.3 mg (a catalytic amount) of tetrakis-triphenylphosphine palladium were added thereto. After the reaction mixture was stirred at 90°C for 18 hours, the solvent was distilled off under reduced pressure. The residue was purified by flash silica gel column chromatography (elution solvent: hexane/ethyl acetate = 5/1) to obtain 310 mg (corresponding to 0.618 mmol) of 6-tributylstannyl-2-(4'-fluorophenyl)imidazo[1,2-a]pyridine (Fig. 6, Step 1).

The NMR measurement results of the resulting 6-tributylstannyl-2-(4'-fluorophenyl)imidazo[1,2-a]pyridine (internal standard: tetramethylsilane) are shown blow.

NMR apparatus employed: JNM-ECP-500 (manufactured by Japan Electron Optics Laboratory Co., Ltd. (JEOL)) $^1$H-NMR (solvent: chloroform-dl, resonance frequency: 500 MHz): δ 7.98 (s, 1H), 7.94-7.90 (m, 2H), 7.77 (s, 1H), 7.60-7.58 (m, 1H), 7.17-7.10 (m, 3H), 1.64-1.48 (m, 6H), 1.35 (sextet, J = 7.3 Hz, 6H), 1.19-1.05 (m, 6H), 0.91 (t, J = 7.3 Hz, 9H).

$^{13}$C-NMR (solvent: chloroform-dl, resonance frequency: 125 MHz): δ 162.7 (d, $^1J_{CF}$ = 246.7 Hz), 145.7, 144.3, 131.4, 130.3 (d, $^4J_{CF}$ = 2.9. Hz), 130.1, 127.7 (d, $^3J_{CF}$ = 8.6 Hz), 122.2, 117.1, 115.6 (d, $^2J_{CF}$ = 21.1 Hz), 106.9, 29.0, 27.3, 13.7, 9.8.

Example 10: Synthesis of [$^{123}$I]-2-(4'-fluorophenyl)-6-iodoimidazo[1,2-a]pyridine

To 60 μL of a mixed solution of 6-tributylstannyl-2-(4'-fluorophenyl)imidazo[1,2-a]pyridine (concentration: 1 mg/mL) in methanol/dimethylsulfoxide (mixing ratio: 9/1), 40 μL of 1 mol/L hydrochloric acid, 15 μL of 1 mmol/L sodium iodide, 12 μL of [$^{123}$I] sodium iodide of 150.1 MBq and 15 μL of 10% (w/v) hydrogen peroxide were added. After the mixed solution was left to stand at 50°C for 10 minutes, It was subjected to HPLC under the following conditions to obtain a fraction of 2-(4'-fluorophenyl)-6-[$^{123}$I]iodoimidazo[1,2-a]pyridine.

EPIC conditions:

Column: Phenomenex Luna C18 (trade name; manufactured by Phenomenex Co.; size: 4.6 x 150 mm)
Mobile phase: 0.1% trifluoroacetic acid/acetonitrile = 20/80 to 0/100 (17 minutes)
Flow rate: 1.0 mL/min.
Detector: Ultraviolet visible absorptiometer (Detection wavelength: 282 nm) and radioactivity counter (manufactured by raytest: type STEFFI)

10 ml of water was added to the fraction. The resulting solution was passed through a Sep-Pak C8 column (trade name:

Sep-Pak (registered trademark) Light C8 Cartridges manufactured by Maters : the packed amount of the packing agent: 130 mg) so that the column adsorbs and collects [$^{123}$I]-2-(4'-fluorophenyl) -6-iodoimidazo[1,2--a]pyridine. The column was rinsed with 1 mL of water, and then 1 mL of diethyl ether was passed therethrough to elute 2-(4'-fluorophenyl)-6-[$^{123}$I]iodoimidazo[1,2-a]pyridine. The amount of radioactivity of the obtained compound was 28.8 MBq at the end of synthesis.

Reference Example 1: Synthesis of [$^{123}$I] -IMPY

[$^{123}$I]-IMPY was synthesized in accordance with the following steps for use in Comparative Examples for evaluations on log$P_{octanol}$ measurement and accumulation in brain.

In accordance with the method described in a literature (Zhi-Ping Zhuang et al., J. Med. Chem., 2003, 46, p.

237-243), 6-tributylstannyl-2-[4'-(N,N-dimethylamino)phenyl]imidazo[1-,2-a]pyridine was synthesized, and dissolved in methanol (concentration: 1mg/mL). To 53 μL of the resulting solution, 75 μL of 1 mol/L hydrochloric acid, 60-70 μL of [$^{123}$I] sodium iodide of 224-253 MBq, 10 μL of a 1 mmol/L sodium iodide solution and 15 of 10% (w/v) hydrogen peroxide were added. After the mixed solution was left to stand at 50˚C for 10 minutes, the solution was subjected to HPLC under the same condition as described, in Example 2, to obtain a fraction of [$^{123}$I]-IMPY.

10 ml of water was added to the fraction. The resulting solution was passed through a reverse phase column (trade name: Sep-Pak (registered trademark) Light C18 Cartridges manufactured by Waters; the packed amount of the packing agent: 130 mg) , so that the column adsorbs and collects the [$^{123}$]-IMPY. The column was rinsed with 1 mL of water, and then 1 mL of diehyl ether was passed therethrough, to elute [$^{123}$I]-IMPY. The obtained radioactivity was 41-57 MBq at the end of synthesis. Further, the TLC analysis was conducted under the same conditions as described in Example 2, and as a result, the radiochemical purity of the compound was 93%.

<u>Examples 11: Measurement of binding to amyloid</u>

Affinity of the present compounds with amyloid was examined by the following in vitro binding tests.

(Method)

(1) A$\beta_{1-42}$ (manufactured by Wako) was dissolved in phosphate buffer (pH 7.4) and shaken at 37˚c for 7.2 hours, to obtain a 1 mg/mL suspension (hereinafter referred to as amyloid suspension in these Examples) of aggregates Aβ (hereinafter referred to as amyloid in this Example).

(2) According to the method described in a literature (Naiki, H., et al., Laboratory Investigation 74, p.374-383 (1996)), the amyloid suspension was subjected to qualitative experiment based on fluorescence spectrophotometric method using Thioflavin T (manufactured by Fluka) to confirm that the aggregates Aβ obtained in (1) was amyloid (measurement conditions: excitation wavelength of 446 nm, and emission wavelength of 490 nm).

(3) A solution in ethanol of [$^{125}$I]-2-(4'-iodophenyl)-6-methoxyimidazo[1,2-a]pyridine synthesized by a method described above in Example 3 (radioactive concentration: 27 MBq/mL) was diluted with a 1% bovine serum albumin-containing phosphate buffer (pH 7.4) to prepare a solution corresponding to 2 nmol/L as a total amount of 2-(4'-iodophenyl)-6-methoxyimidazo[1,2-a]pyridine.

(4) To each well of a 96-well microplate, 50 μL of a solution prepared above in (3) and 50 μL of a solution obtained by dissolving amyloid suspension in 1 % bovine serum albumin-containing phosphate buffer (pH 7.4) (where amyloid concentration was adjusted in accordance with amyloid concentration in a sample solution) were added, and then 150 μL of 1 % bovine serum albumin-containing phosphate buffer (pH 7.4) was added to prepare solutions containing amyloid at final concentrations of 25, 62.5, 250, 625 and 1000 nmol/L (corresponding to 400 pmol/L as a total amount of 2-(4'-iodophenyl)-6-methoxyimidazo[1,2-a]pyridine).

(5) The microplate was shaken at a given rate (400 rpm) at 22˚C for 3 hours. Then, a mined solution of each well was filtered through a glass fiber filter (trade name: Mulutiscreen™-FC, manufactured by Millipore), to separate [$^{125}$I]-2-(4'-iodophenyl)-6-methoxyimidazo[1,2-a]pyridine attached to amyloid from free [$^{123}$I] -2-(4'-iodophenyl)-6-methoxyimidazo [1,2-a]pyridine.

(6) The glass fiber filter after used for filtration of the sample solution was washed with a 1 % bovine serum albumin-containing phosphate buffer (pH 7.4) (0.5 mL x 5), and then radioactive count (hereinafter referred to as A) of the glass fiber filter was measured with an autowell gamma system (manufactured by Aloka, Type: ARC-301B).

(7) Separately, a solution in methanol of 2-(4'-iodophenyl)-6-methoxyimidazo[1,2-a]pyridine (non-radioactive iodinated form) prepared in Example 4 was used to perform the same procedure as described above in (3) to prepare a solution of 2-(4'-iodophenyl)-6-methoxyimidazo[1,2-a]pyridine at a concentration of 15 μmol/L. 50 μL of this solution was added two each well of a 96-well microplate with 50 μL of a solution prepared above in (3) and 50 μL of a solution obtained by dissolving amyloid suspension in 1 % bovine serum albumin-containing phosphate buffer (pH 7. 4) (where amyloid concentration was adjusted in accordance at an amyloid concentration in a sample solution) and 100 μL of 1 % bovine serum albumin-containing phosphate buffer (pH 7.4) were added to prepare solutions containing amyloid at final concentrations of 25, 62.5, 250, 625 and 1000 nmol/L. The same procedures as in the above (5) and (6) were carried out to measure the radioactive count remained in the glass fiber filter (hereinafter referred to as B) in this microplate.

(8) Using the values of the radioactive counts determined above in (6) and (7), the radioactive count of [$^{123}$I]-2-(4'-iodophenyl)-6-methoxyimidazo[1,2-a]pyridine specifically binding to amyloid was calculated in accordance with the following formula (1), and a relation with the addition amount of amyloid was evaluated.

$$\textit{Radioactive count} = A - B \qquad (1)$$

(Results)

A relation between the radioactive count calculated above in (8) and the concentration of amyloid in the sample solution is shown in Fig. 5. As shown in this figure, the value of the radioactive count calculated above in (8) showed a tendency to increase with increment of the concentration of amyloid. On the conditions of the present experiment, amyloid and the compound attached to amyloid are retained by the glass fiber. The radioactivity remained on the glass fiber without binding to amyloid was substantially removed from the value of radioactive count calculated above in (8). Thus, the radioactive count on the glass fiber measured in the present example can be said as a value reflecting the amount of the compound specifically attached to amyloid. Therefore, the fact that the value of the radioactive count determined above in (8) was increased with increment of the concentration of amyloid, is the result strongly suggesting that [$^{123}$I]-2-(4'-iodophenyl)-6-methoxyimidazo[1,2-a]pyridine is a compound capable of binding to amyloid.
From the above-mentioned results, it has been indicated that [$^{125}$I]-2-(4'-iodophenyl)-6-methoxyimidazo[1,2-a]pyridine is capable of binding to amyloid.

<u>Example 12-14, Comparative Example 1: Measurement of partition coefficient based on the octanol extraction method</u>

Partition coefficients based on the octanol extraction method (hereinafter referred to as $\log P_{octanol}$) were measured, which are generally known as an indicator of permeability of compounds through the blood-brain barrier (hereinafter referred to as BBB).

(Method)

A diethyl ether solution of [123$^{I}$]-6-iodo-2-(4'-methoxyphenyl)imidazo[1,2-a]pyridine prepared in Example 7 (Example 12), a diethyl ether solution of [$^{123}$I]-2-(4'-iodophenyl)-6-methoxyimidazo[1,2-a]pyridine prepared in Example 2 (Example 13), a diethyl ether solution of [$^{123}$I]-2-(4'-fluorophenyl)-6-iodoimidazo[1,2-a]pyridine prepared in Example 10 (Example 14) and a diethyl ether solution of [$^{123}$I] -IMFY prepared in Reference Example 1 (Comparative Example 1) were each diluted with a 10 mg/ml ascorbic acid-containing physiological saline solution and adjusted to a radioactive concentration of 20-30 HBq/mL. To 2 mL of octanol, 10 $\mu$L each of the prepared sample solutions was respectively added, and 2 mL of a 10 mmol/L phosphate buffer (pH 7.4) was further added, followed by stirring for 30 seconds. After the mixture was centrifuged with a low-speed centrifuge (2000 rpm x 60 min.), the octanol layer and the water layer were sampled each in an amount of 1 mL, and subjected to measurement of radioactivity count with an autowell gamma system (Type: ARC-301B, manufactured by Aloka). Using the obtained radioactivity count, $\log P_{octanol}$ was calculated in accordance with the equation (2).

$$\log P_{octanol} = \log_{10}\left(\frac{Radioactivity\ count\ of\ octanol\ layer}{Radioactivity\ count\ of\ water\ layer}\right) \cdots (2)$$

(Results)

The results are shown in Table 1. As shown in this table, all the compounds showed $\log P_{octanol}$ values between 1 and 3. It is known that compounds permeable to BBB show a $\log P_{octanol}$ value between 1 and 3 (Douglas D. Dischino et al., J. Nucl. Med., (1983) 24, p.1030-1033) From the above results, it is implied that all the compounds have a BBB permeability comparable to IMPY.

Table 1. $\log P_{octanol}$ value of the present compound

| Experiment | Compound | $\log P_{octanol}$ value |
|---|---|---|
| Comparative Example 1 | [$^{123}$I]-IMPY | 1.9 |
| Example 12 | [$^{123}$I]-6-iodo-2-(4'-methoxyphenyl)imidazol[1, 2-a]pyridine | 1.7 |
| Example 13 | [$^{123}$I]-2-(4'-iodophenyl)-6-methoxyimidazo[1, 2-a]pyridine | 2.3 |
| Example 14 | [$^{123}$I]-(4'-fluorophenyl)-6-iodoimidazo[1,2-a]pyridine | 2.3 |

Example 15-16, Comparative Example 2: Measurement of transferability into brain and clearance

Using [$^{123}$I]-2-(4'iodophenyl)-6-methoyimidazo[1,2-a] pyridine and [$^{123}$I]-2-(4'-fluorophenyl)-6-iodoimidazo[1,2-a pyridine, a time course change of radioactive accumulation in brain of male Wistar rats (7-week old) was measured.

(Method)

0.05 mL (20-30 MBq/mL in radioactive concentration) of a solution of [$^{123}$I]-2-(4'-iodophenyl)-6-methoxyimidazo [1,2-a] pyridine prepared above in Example 2 (Example 15) in a 10 mg/mL ascorbic acid-containing physiological saline solution and a solution of [$^{123}$I]-2-(-4'-fluorophenyl)-6-idoimidazo[1,2-a]pyridine prepared above in Example 10 (Example 16) in a 10 mg/mL ascorbic acid-containing physiological saline solution were each injected under thiopental anesthesia into the tail vein of the mail Wistar rats (7-week old). The rats where sacrificed bey bleeding from abdominal artery, and brains were removed and subjected to measurement of mass of brains and further subjected to measurement of radioactivity (hereinafter referred to as A in this Example) with a single channel analyzer (detector type: SP-20 manufactured, by OHYO KOKEN KOGYO Co., Ltd.) 2, 5, 30 and 60 minutes after the injection. Also, radioactivity (hereinafter referred to as B in this Example) of the remain of the whole body was measured in the same manner as above. Using these measurement results, radioactive accumulation per unit weight off brain (%ID/g) at the respective time points was calculated in accordance with the following formula (3) (Examples 15 and 16).
Separately, a solution (20-30 MBq/mL in radioactive concentration) of [$^{123}$I]-IMPY prepared above in Reference Example 1 in a 10 mg/mL ascorbic acid-containing physiological saline solution was prepared. The same procedures as above were performed to calculate radioactive accumulation per unit wight of brain (%ID/g) at the respective time points (Comparative Example 2).
Three animals were used for Example 15, Example 16 and Reference Example 2 at the respective time points.

$$\%ID/g = \frac{\mathrm{A}}{\mathrm{B} \times brain\ weight} \times 100 \quad \cdots (3)$$

(Result)

The results are shown in Table 2. As shown in Table 2, [$^{123}$I] -2-(4'-iodophenyl)-6-methoxyimidazo[1,2-a] pyridine (Example 15) and [$^{123}$I]-2-(4'-fluorophenyl)-6-iodoimidazo[1,2-a]pyridine (Example 16) showed an accumulation comparative to [$^{123}$I] -IMPY (Comparative Example 2) at the time point of two minutes after the injection, and they showed a tendency to rapidly clear away in 60 minutes. These results suggest that [$^{123}$I]-2-(4'-iodophenyl)-6-methoxyimidazo [1,2-a] pyridine and [$^{123}$I]-2-(4'-fluorophenyl)-6-iodoimidazo[1,2-a]pyridine possess excellent transferability to brain and rapid clearance from brain comparable to [$^{123}$I]-IMPY.

Table 2: Radioactive accumulation in brain, of the present compound after intravenous injection (rats)

| | Radioactive accumulation per unit weight (%ID/g) | | | |
|---|---|---|---|---|
| | After 2 min. | After 5 min. | After 30 min. | After 60 min. |
| Example 15 | 1.26 | 0.89 | 0.19 | 0.09 |
| Example 16 | 1.00 | 0.72 | 0.12 | 0.05 |
| Comparative Example 2 | 1.19 | 0.97 | 0.23 | 0.09 |

Example 17: Confirmation of imaging of amyloid in brain by [$^{123}$I]-6-iodo-2- (4'-methoxyphenyl) imidazo[1,2-a] pyridine

The following experiment was carried out in order to examine whether amyloid in brain can be imaged by the compound, of the present invention.

(Method)

(1) $A\beta_{1-42}$ (manufactured by Wako) was dissolved in phosphate buffer (pH 7.4) and shaken at 37˚C for 72 hours, to obtain a 1 mg/mL suspension of aggregated Ap (hereinafter referred to as amyloid suspension in this Example).

(2) 2.5 μL (corresponding to 25 μg) of the amyloid suspension was injected under thiopental into an amygdaloid nucleus on one side of a male Wistar rat (7-week old) . As a control, 2.5 μL of a phosphate buffered physiological saline solution (pH 7.4) was injected at amygdaloid nucleus on the other side of the rat. The rats were examined 1 day after the injection of the amyloid suspension and the phosphate buffered physiological saline solution (pH 7.4).

(3) [$^{123}$I]-6-iodo-2-(4'-methoxyphenyl)imidazo[1,2-a] pyridine was dissolved in a 10mg/mL ascorbic acid-containing physiological saline solution to obtain a sample solution (32 MBq/mL in radioactivity concentration). This sample solution was injected under thiopental anesthesia into the rat through the tail vein (dosage: 0.5 mL, dosed radioactivity: 16 MBq equivalent) .

(4) Brain was removed 60 minutes after the injection to prepare a brain slice of 10 μm in thickness with a microtome (type: CM3050S, manufactured by LEIGA). The brain slice was exposed to an imaging plate for 20 hours, and then image analysis was carried out by use of a Bio-imaging Analyzer (type: BAS-2500; manufactured by FUJIFILM Corporation).

(5) After the completion of the image analysis using the Bio-imaging Analyzer, pathological staining with Thioflavin T was carried out to perform imaging by use of a fluorescence microscope (manufactured by NIKON Corporation; type: TE2000-U model; excitation wavelength: 400-440 nm; detection wavelength: 470 nm). Thus, it was confirmed that amyloid was deposited on the slice (Fig. 8b).

(Result)

Fig. 8 shows images by autoradiogram and Thioflavin T staining of the brain slice of the rat to which amyloid was injected intracerebrally. As shown in this figure, a marked accumulation of radioactivity was observed in the amygdaloid nucleus on the side to which the amyloid suspension was injected. From the result of Thioflavin T staining in the site where radioactivity is accumulated, it was confirmed that amyloid was present in the accumulation, site. On the other hand, no significant accumulation of radioactivity was observed in the amygdaloid nucleus on the side to which the physiological saline solution was injected, compared with the other sites. These results suggest that [$^{123}$I]-6-iodo-2-(4'-methoxyphenyl) imidazo [1,2-a] pyridine possesses a property of accumulating on intracerebral amyloid and a capability of imaging intracerebral amyloid.

Example 18: Confirmation of imaging of amyloid in brain by [$^{123}$I]-2-(4'-iodophenyl)-6-methoxymidazo[1,2-a] pyridine

The following experiment was carried out in order to examine whether amyloid in brain can be imaged by the compound of the present invention.

(Method)

The same procedures as in Example 17 were performed except using a solution of [$^{123}$I] -2-(4'-iodophenyl)-6-methoxyimidazo[1,2-a] pyridine in a 10 mg/mL ascorbic acid-containing physiological saline solution as a sample solution (radioactive concentration: 20 MBq/mL, and a capability of imaging intracerebral amyloid of [$^{123}$I]-2-(4'-iodophenyl)-6-methoxyimidazo[1,2-a] pyridine was observed (dosage: 0.5 mL, dosed radioactivity: 10 MBq equivalent).

Fig. 9 shows images by autoradiogram and Thioflavin T staining of the brain slice of the rat to which amyloid was injected intracerebrally. As shown in this figure, a marked accumulation of radioactivity was observed in the amygdaloid nucleus on the side to which the amyloid suspension was injected. From the result of Thioflavin T staining in the site where radioactivity is accumulated, it was confirmed that amyloid was present in the accumulation site. On the other hand, no significant accumulation of radioactivity was observed in the amygdaloid nucleus on the side to which the physiological saline solution was injected, compared with the other site. These results suggest that [$^{123}$I]-2-(4'-iodophenyl)-6-methoxyimidazo[1,2-a]pyridine possesses a property of accumulating on intracerebral amyloid and a capability of imaging intracerebral amyloid.

Example 19: Confirmation of imaging of amyloid in brain by [$^{123}$I]-2-(4'-fluorophenyl)-6-iodoimidazo[1,2-a] pyridine

The following experiment was carried out in order to examine whether amyloid in brain can be imaged by the compound of the present invention.

(Method)

The same procedures as in Example 17 were performed except using a solution of [$^{123}$I]-2-(4'-fluorophenyl)-6-iodoimidazo [1,2-a]pyridine in a 10 mg/mL ascorbic acid-containing physiological saline solution as a sample solution

(radioactive concentration: 24 MBq/mL), and a capability of imaging intracerebral amyloid of [$^{123}$I]-2-(4'fluorophenyl)-6-iodoimidazo[1,2-a]pyridine was observed (dosage: 0.5 mL, dosed radioactivity: 12 MBq equivalent).

(Result)

Fig. 10 shows images by autoradiogram and Thioflavin T staining of the brain slice of the rat to which amyloid was injected intracerebrally. As shown in this figure, a marked accumulation of radioactivity was observed in the amygdaloid nucleus on the side to which the amyloid suspension was injected. From the result of Thioflavin T staining in the site where radioactivity is accumulated, it was confirmed that amyloid was present in the accumulation site. On the other hand, no significant accumulation of radioactivity was observed in the amygdaloid nucleus on the side to which the physiological saline solution was injected, compared with the other sites. These results suggest that [$^{123}$I]-2-(4'-flurophenyl)-6-iodoimidazo [1,2-a]pyridine possesses a property of accumulating on intracerebral amyloid and a capability of imaging intracerebral amyloid.

Example 20: Measurement of transferability into brain and clearance

Using [$^{123}$I]-6-iodo-2-(4'-methoxyphenyl)imidazo[1,2-a]pyridine, a time course change of radioactive accumulation in brain of male Wistar rats (7-week old) was measured.

(Method)

A time course change of radioactive accumulation in brain of the above rat was measured by performing the same procedure as in Examples 15 and 16 except using a solution of [$^{123}$I]-6-iodo-2-(4'-methoxyphenyl)imidazo[1,2-a]pyridine prepared above in Example 7 in a 10 mg/mL ascorbic acid-containing physiological saline solution as a sample solution (radioactive concentration: 20-30 MBq/mL).

(Result)

The results are shown in Table 3. As shown in Table 3, [$^{123}$I] -6-iodo-2-(4'-methoxyphenyl)imidazo[1,2-a]pyridine showed an accumulation comparable to [$^{123}$I]-IMPY (referred to the above Reference Example 2) at the time point of two minutes after the injection, and then showed a tendency to rapidly clear away in it minutes. These results suggest that [$^{123}$I]-6-iodo-2-(4'-methoxyphenyl)imidazo[1,2-a]pyridine possesses excellent transferability to brain and rapid clearance from brain similarly to $^{123}$I-IMPY.

Table 3: Radioactive accumulation in brain of the present compound after intravenous injection (rats)

| | Radioactive accumulation per unit weight (%ID/g) | | | |
|---|---|---|---|---|
| | After 2 min. | After 5 min. | After 30 min. | After 60 min. |
| Example 20 | 1.04 | 0.72 | 0.12 | 0.04 |

INDUSTRIAL APPLICABILITY

The compounds of the present invention can be utilized in the field of diagnostic agents.

BRIEF DESCRIPTION OF THE DRAWINGS

Fig. 1 is a scheme of synthesis of 2-(4'-tributylstannylphenyl)-6-methoxyimidazo[1,2-a]pyridine.
Fig. 2 is a scheme of synthesis of 2-(4'-iodophenyl)-6-methoxyimidazo[1,2-a]pyridine.
Fig. 3 is a scheme of synthesis of 6-iodo-2-(4'-methoxyphenyl)imidazo[1,2-a]pyridine.
Fig. 4 is a scheme of synthesis of 6-tributylstannyl-2-(4'-methoxyphenyl)imidazo[1,2-a]pyridine.
Fig. 5 is a scheme of synthesis of 2-(4'-fluorophenyl)-6-iodoimidazo[1,2-a]pyridine.
Fig. 6 is a scheme of synthesis of 6-tributylstannyl-2-(4'-fluorophenyl)imidazo[1,2-a]pyridine.
Fig. 7 is a relationship between amyloid concentration and radioactive concentration in sample solutions.

Fig. 8 (a) is an autoradiogram of the brain slice after the injection of [$^{123}$I]-6-iodo-2-(4'-methoxyphenyl)imidazo[1,2-a]pyridine, and Fig. 8 (b) is a fluorescent microscopic image of the Thioflavin T stained sample (a magnification of the site to which the amyloid suspension was injected).
Fig. 9 (a) is an autoradiogram of the brain slice after the injection of [$^{123}$I]-2-(4'-iodophenyl)-6-methoxyimidazo[1,2-a]pyridine, and Fig. 9 (b) is a fluorescent microscopic image of the Thioflavin T stained sample (a magnification of the site to which the amyloid suspension was injected).
Fig. 10 (a) is an autoradiogram of the brain slice after the injection of [$^{123}$I]-2-(4'-fluorophenyl)-6-iodoimidazo[1,2-a] pyridine, and Fig. 10 (b) is a fluorescent microscopic image of the Thioflavin T stained sample (a magnification of the site to which the amyloid suspension was injected).

## Claims

**1.** A compound represented by the following formula (1), or a salt thereof:

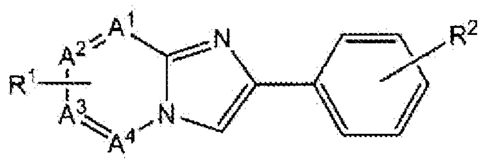

(1)

wherein $A^1$, $A^2$, $A^3$ and $A^4$ independently represents a carbon or nitrogen,
$R^1$ is a group selected from the group consisting of hydrogen, hydroxyl group, carboxyl group, sulfuric acid group, amino group, nitro group, cyano group, non-radioactive halogen substituents, alkyl substituents with 1-4 carbon atoms and alkoxy substituents with 1-4 carbon atoms, and $R^2$ is a radioactive halogen substituent,
provided that at least one of $A^1$, $A^2$, $A^3$ and $A^4$ represents a carbon, and $R^1$ binds to a carbon represented by $A^1$, $A^2$, $A^3$ or $A^4$.

**2.** A compound or a salt thereof according to claim 1, wherein at least three of $A^1$, $A^2$, $A^3$ and $A^4$ represent carbons.

**3.** A compound or a salt thereof according to claim 2, wherein all of $A^1$, $A^2$, $A^3$ and $A^4$ represent carbons.

**4.** A compound or a salt thereof according to any one of claims 1 to 3, wherein $R^2$ is a radioactive halogen substituent selected from the group consisting of $^{18}$F, $^{76}$Br, $^{123}$I, $^{124}$I, $^{125}$I and $^{131}$I.

**5.** A compound represented by the following formula (2), or a salt thereof:

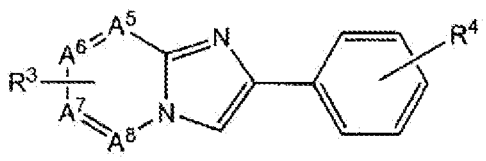

(2)

wherein $A^5$, $A^6$, $A^7$ and $A^8$ independently represents a carbon or nitrogen,
$R^3$ is a radioactive halogen substituent, and
$R^4$ is a group selected from the group consisting of hydrogen, carboxyl group, sulfuric acid group, nitro group, cyano group, non-radioactive halogen substituents, alkyl substituents with 1-4 carbon atoms and methoxy substituents, provided that at least one of $A^5$, $A^6$, $A^7$ and $A^8$ represents a carbon, and $R^3$ binds to a carbon represented by $A^5$, $A^6$, $A^7$ or $A^8$.

**6.** A compound or a salt thereof according to claim 5, wherein at least three of $A^5$, $A^6$, $A^7$ and $A^8$ represent carbons.

**7.** A compound or a salt thereof according to claim 6, wherein all of $A^5$, $A^6$, $A^7$ and $A^8$ represent carbons.

**8.** A compound or a salt thereof according to any one of claims 5 to 7, wherein $R^3$ is a radioactive halogen substituent selected from the group consisting of $^{18}F$, $^{76}Br$, $^{123}I$, $^{124}I$, $^{125}I$ and $^{131}I$.

**9.** A compound represented by the following formula (3), or a salt thereof:

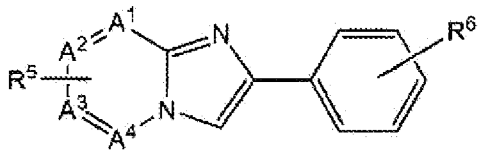

(3)

wherein $A^1$, $A^2$, $A^3$ and $A^4$ independently represents a carbon or nitrogen,
$R^5$ is a group selected from the group consisting of hydrogen, hydroxyl group, carboxyl group, sulfuric acid group, amino group, nitro group, cyano group, non-radioactive halogen substituents, alkyl substituents with 1-4 carbon atoms and alkoxy substituents with 1-4 carbon atoms, and $R^6$ is a group selected from the group consisting of a non-radioactive halogen substituent, nitro group, trialkylstannyl substituent having alkyl chains with 1 to 4 carbon atoms, triphenylstannyl group and trialkylammonium group having alkyl chains with 1 to 4 carbon atoms, provided that at least one of $A^1$, $A^2$, $A^3$ and $A^4$ represents a carbon, and $R^5$ binds to a carbon represented by $A^1$, $A^2$, $A^3$ or $A^4$.

**10.** A compound or a salt thereof according to claim 9, wherein at least three of $A^1$, $A^2$, $A^3$ and $A^4$ represent carbons.

**11.** A compound or a salt thereof according to claim 10, wherein all of $A^1$, $A^2$, $A^3$ and $A^4$ represent carbons.

**12.** A compound represented by the following formula (4), or a salt thereof:

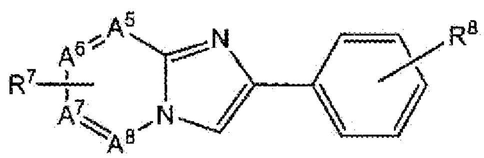

(4)

wherein $A^5$, $A^6$, $A^7$ and $A^8$ independently represents a carbon or nitrogen,
$R^7$ is a group selected from the group consisting of a non-radioactive halogen substituent, nitro group, trialkylstannyl substituent having alkyl chains with 1 to 4 carbon atoms, triphenylstannyl group and trialkylammonium group having alkyl chains with 1 to 4 carbon atoms, and
$R^8$ is a group selected from the group consisting of hydrogen, carboxyl group, sulfuric acid group, nitro group, cyano group, non-radioactive halogen substituents, alkyl substituents with 1-4 carbon atoms and methoxy substituent, provided that at least one of $A^5$, $A^6$, $A^7$ and $A^8$ represents a carbon, and $R^7$ binds to a carbon represented by $A^5$, $A^6$, $A^7$ or $A^8$.)

**13.** A compound or a salt thereof according to claim 12, wherein at least three of $A^5$, $A^6$, $A^7$ and $A^8$ represent carbons.

**14.** A compound or a salt thereof according to claim 13, wherein all of $A^5$, $A^6$, $A^7$ and $A^8$ represent carbons.

**15.** A diagnostic agent for Alzheimer's disease, which comprises a compound represented by the following formula (1), or a salt thereof:

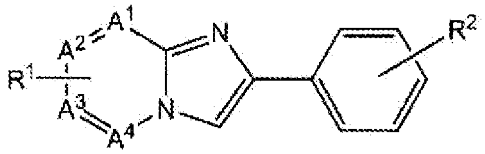

(1)

wherein $A^1$, $A^2$, $A^3$ and $A^4$ independently represents a carbon or nitrogen,
$R^1$ is a group selected from the group consisting of hydrogen, hydroxyl group, carboxyl group, sulfuric acid group,

amino group, nitro group, cyano group, non-radioactive halogen substituents, alkyl substituents with 1-4 carbon atoms and alkoxy substituents with 1-4 carbon atoms, and $R^2$ is a radioactive halogen substituent,
provided that at least one of $A^1$, $A^2$, $A^3$ and $A^4$ represents a carbon, and $R^1$ binds to a carbon represented by $A^1$, $A^2$, $A^3$ or $A^4$.

**16.** The diagnostic agent for Alzheimer's disease according to claim 15, wherein at least three of $A^1$, $A^2$, $A^3$ and $A^4$ represent carbons.

**17.** The diagnostic agent for Alzheimer's disease according to claim 16, wherein all of $A^1$, $A^2$, $A^3$ and $A^4$ represent carbons.

**18.** The diagnostic agent for Alzheimer's disease according to any one of claims 15 to 17, wherein $R^2$ is a radioactive halogen substituent selected from the group consisting of $^{18}F$, $^{76}Br$, $^{123}I$, $^{124}I$, $^{125}I$ and $^{131}I$.

**19.** A diagnostic agent for Alzheimer's disease, which comprises a compound represented by the following formula (2), or a salt thereof:

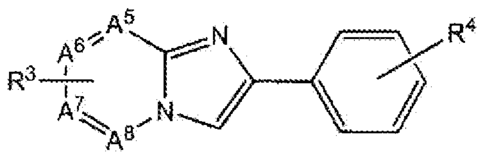

(2)

wherein $A^5$, $A^6$, $A^7$ and $A^8$ independently represents a carbon or nitrogen,
$R^3$ is a radioactive halogen substituent, and
$R^4$ is a group selected from the group consisting of hydrogen, carboxyl group, sulfuric acid group, nitro group, cyano group, non-radioactive halogen substituents, alkyl substituents with 1-4 carbon atoms and alkoxy substituents with 1-4 carbon atoms, provided that at least one of $A^5$, $A^6$, $A^7$ and $A^8$ represents a carbon, and $R^3$ binds to a carbon represented by $A^5$, $A^6$, $A^7$ or $A^8$.

**20.** The diagnostic agent for Alzheimer's disease according to claim 19, wherein at least three of $A^5$, $A^6$, $A^7$ and $A^8$ represent carbons.

**21.** The diagnostic agent for Alzheimer's disease according to claim 20, wherein all of $A^5$, $A^6$, $A^7$ and $A^8$ represent carbons.

**22.** The diagnostic agent for Alzheimer's disease according to any one of claims 19 to 21, wherein $R^3$) is a radioactive halogen substituent selected from the group consisting of $^{18}F$, $^{76}Br$, $^{123}I$, $^{124}I$, $^{125}I$ and $^{131}I$.

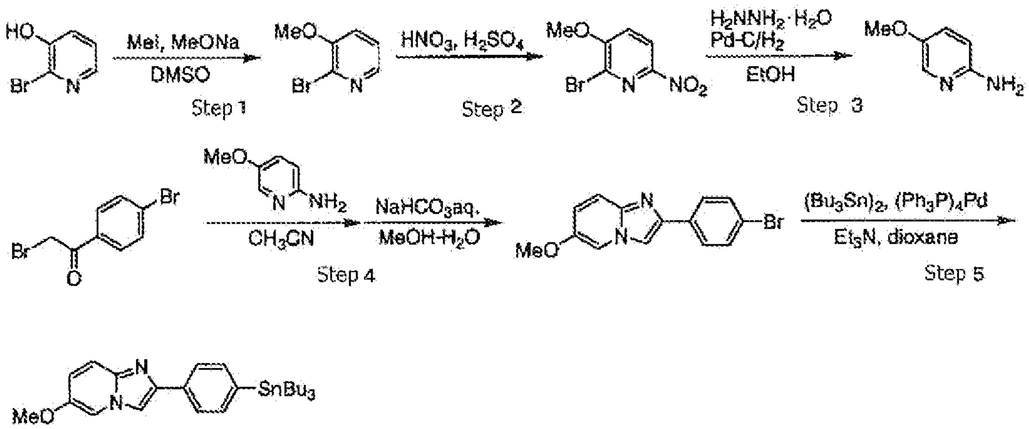


Figure 1

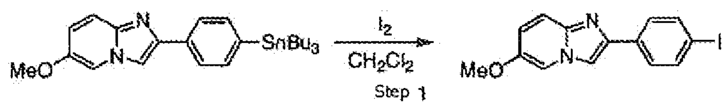


Figure 2

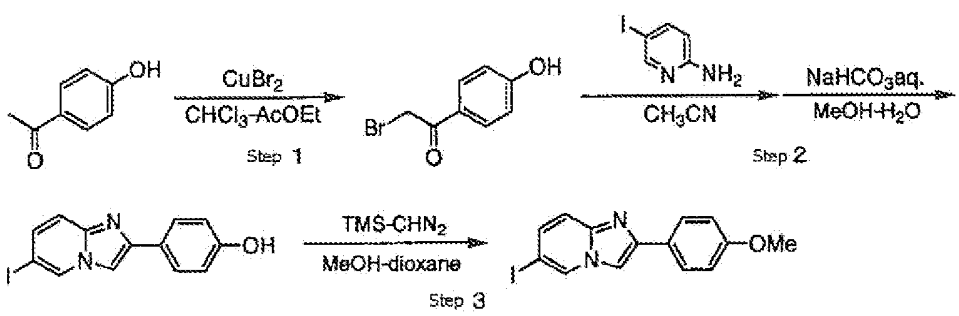


Figure 3

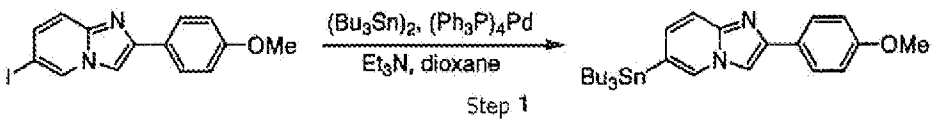


Figure 4

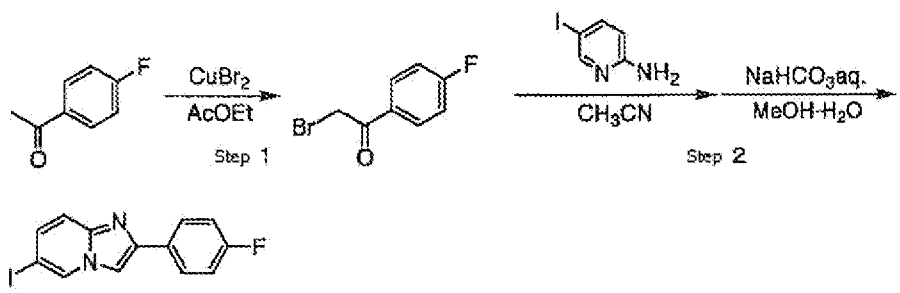


Figure 5

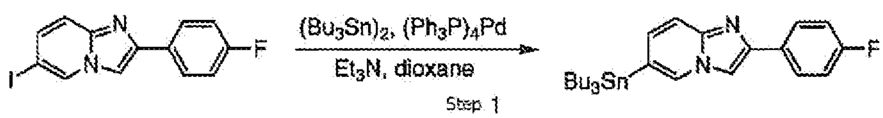


Figure 6

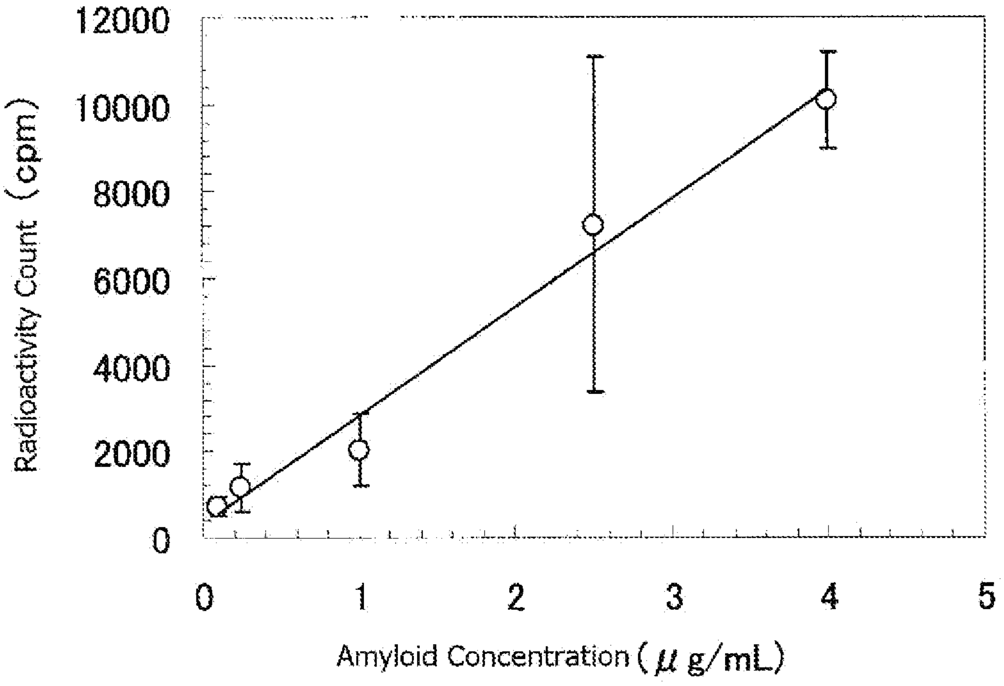


Figure 7

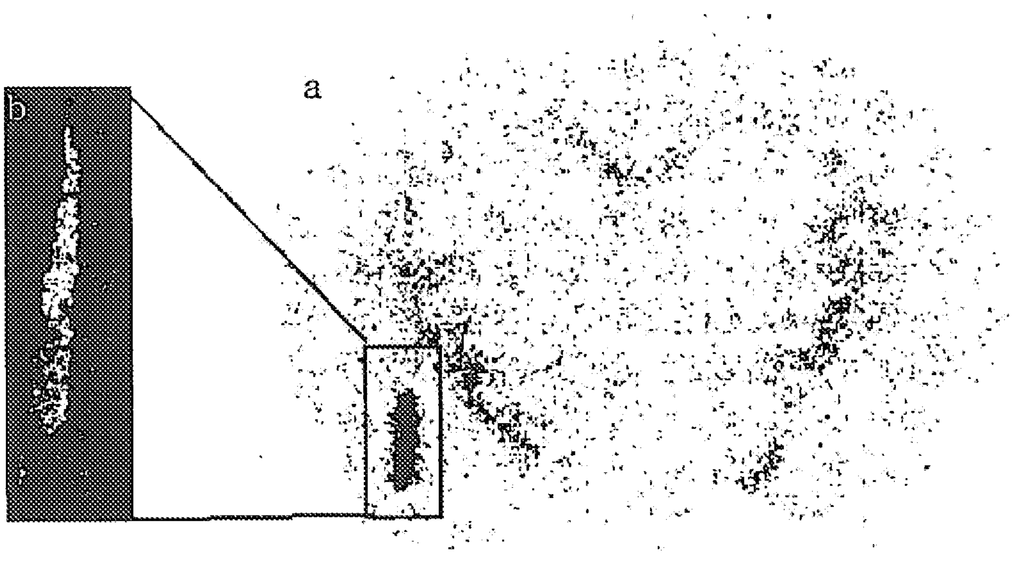


Figure 8

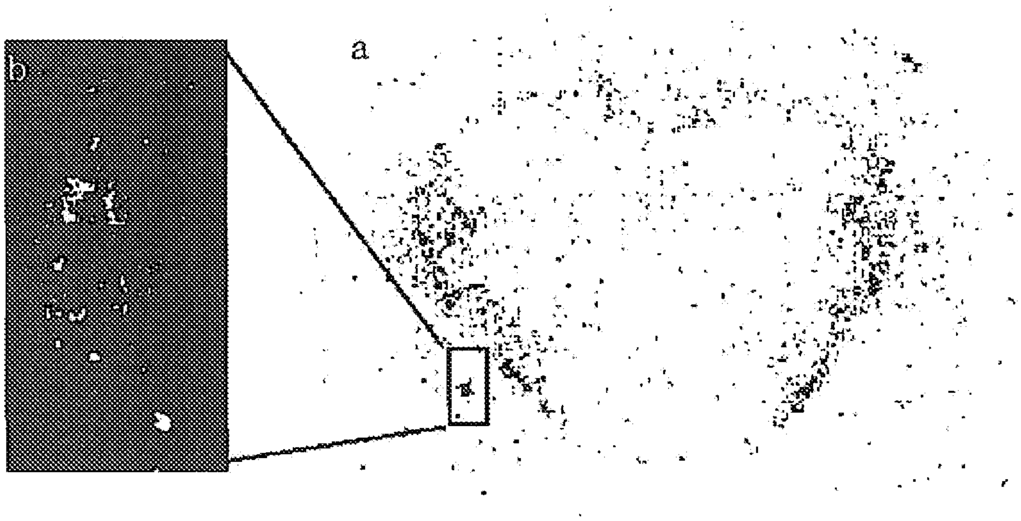


Figure 9

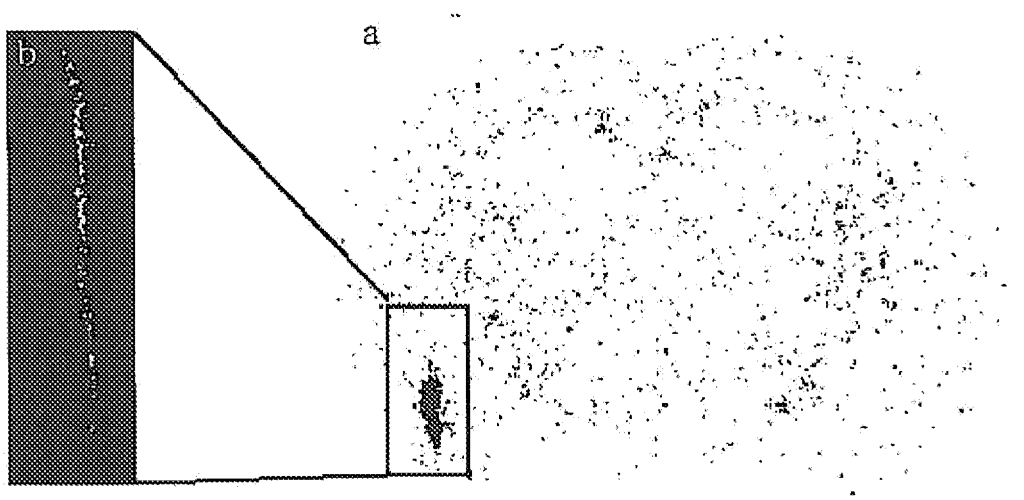


Figure 10

**INTERNATIONAL SEARCH REPORT**

International application No.
PCT/JP2007/071121

A. CLASSIFICATION OF SUBJECT MATTER
*C07D471/04*(2006.01)i, *A61K51/00*(2006.01)i

According to International Patent Classification (IPC) or to both national classification and IPC

B. FIELDS SEARCHED

Minimum documentation searched (classification system followed by classification symbols)
C07D471/04, A61K51/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus(STN), REGISTRY(STN), MARPAT(STN)

C. DOCUMENTS CONSIDERED TO BE RELEVANT

| Category* | Citation of document, with indication, where appropriate, of the relevant passages | Relevant to claim No. |
|---|---|---|
| X<br>A | LU Chun-Xiong et al., Synthesis and biodistribution of [131I]IMPY, Nuclear Science and Techniques, 2005, Vol.16, No.5, p.289-292 | 5-14,19-22<br>1-4,15-18 |
| X<br>A | SUNDBERG, R. J. et al., Preparation of 2-Aryl and 2-Aryloxymethyl Imidazo[1, 2-a]pyridines and Related Compounds, Journal of Heterocyclic Chemistry, 1988, Vol.25, No.1, p.129-137 | 9-14<br>1-8,15-22 |
| X<br>A | WO 2004/087641 A1 (Daiichi Pharmaceutical Co., Ltd.),<br>14 October, 2004 (14.10.04),<br>Referential examples 46, 170, 172, 175<br>& EP 1612204 A1 & US 2006/0276433 A1 | 9-14<br>1-8,15-22 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
22 November, 2007 (22.11.07)

Date of mailing of the international search report
04 December, 2007 (04.12.07)

Name and mailing address of the ISA/
Japanese Patent Office

Facsimile No.

Authorized officer

Telephone No.

Form PCT/ISA/210 (second sheet) (April 2007)

**INTERNATIONAL SEARCH REPORT**

International application No.
PCT/JP2007/071121

C (Continuation). DOCUMENTS CONSIDERED TO BE RELEVANT

| Category* | Citation of document, with indication, where appropriate, of the relevant passages | Relevant to claim No. |
|---|---|---|
| X<br>A | LANGE, J. et al., A structure-activity relationship study of the affinity of selected imidazo[1, 2-a]pyridine derivatives, congeners of zolpidem, for the omega1-subtype of the benzodiazepine receptor, Acta Poloniae Pharmaceutica-Drug Research, 2001, Vol.58, No.1, p.43-52 | 9-14<br>1-8,15-22 |
| X<br>A | JP 2005-500315 A (SmithKline Beecham Corp.), 06 January, 2005 (06.01.05), Examples<br>& WO 2003/000689 A1 & EP 1401836 A1<br>& US 2005/0228004 A1 | 9-14<br>1-8,15-22 |
| X<br>A | JP 2005-508955 A (SmithKline Beecham Corp.), 07 April, 2005 (07.04.05), Examples<br>& WO 2003/031446 A1 & EP 1432712 A1<br>& US 2004/0248917 A1 | 9-14<br>1-8,15-22 |
| X<br>A | HERVET, M. et al., Comparative study on the reactivity of 6-haloimidazo[1, 2-a]pyridine derivatives towards Negishi- and Stille-coupling reactions, Helvetica Chimica Acta, 2003, Vol.86, No.10, p.3461-3469 | 9-14<br>1-8,15-22 |
| X<br>A | WO 2005/043630 A1 (Idemitsu Kosan Co., Ltd.), 12 May, 2005 (12.05.05), Page 48<br>& EP 1679747 A1 & US 2007/0120111 A1 | 9-14<br>1-8,15-22 |
| X<br>A | WO 2005/086808 A2 (THE UNIVERSITY OF NORTH CAROLINA ATCHAPELHILL), 22 September, 2005 (22.09.05), Scheme 3 and 4<br>& EP 1745045 A2 & US 2005/0282853 A1 | 9-14<br>1-8,15-22 |
| X<br>A | KATSIFIS, A. et al., Synthesis of [123I]iodine labeled imidazo[1, 2-b]pyridazines as potential probes for the study of peripheral benzodiazepine receptors using SPECT, Radiochimica Acta, 2004, Vol.92, No.4-6, p.305-309 | 9,10,12,13<br>1-8,11,14-22 |
| X<br>A | BARLIN, G. B., Imidazo[1, 2-b]pyridazines: Syntheses and interaction with central and peripheral-type (mitochondrial) benzodiazepine receptors, Journal of Heterocyclic Chemistry, 1998, Vol.35, No.5, p.1205-1217 | 9,10,12,13<br>1-8,11,14-22 |

Form PCT/ISA/210 (continuation of second sheet) (April 2007)

**INTERNATIONAL SEARCH REPORT**

International application No.
PCT/JP2007/071121

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2004-525192 A (F. Hoffmann-La Roche AG.), 19 August, 2004 (19.08.04), Examples<br>& WO 2002/092086 A1 & EP 1381363 A1<br>& US 2002/0188128 A1 | 9-11<br>1-8,12-22 |
| X<br>A | KATSIFIS, A. et al., Synthesis of [123I]N', N'-Dimethyl-6-methyl-(4'-iodophenyl)imidazo [1, 2-a] pyridine-3-acetamide for the study of peripheral benzodiazepine receptors using SPECT, Journal of Labelled Compounds and Radiopharmaceuticals, 2000, Vol.43, No.4, p.385-394 | 9-11<br>1-8,12-22 |
| X<br>A | BARLIN, G. B. et al., Imidazo[1, 2-b] pyridazines. XX Syntheses of some 3-acylaminomethyl-6-(chloro, fluoro, methoxy, methylthio, phenoxy and phenylthio)-2-(phenyl, 4-t-butylphenyl, 4-cyclohexylphenyl, beta-naphthyl and styryl)imidazo[1, 2-b] pyridazines and their interaction with central and peripheral-type benzodiazepine receptors, Australian Journal of Chemistry, 1996, Vol.49, No.4, p.451-461 | 12-14<br>1-11,15-22 |
| X<br>A | Danqian XU et al., Synthesis of 2-arylimidazo [1, 2-a]pyrimidines by the Chichibabin synthesis in ionic liquids, Journal of Chemical Research, Synopses, 2003, No.10, p.645-647 | 9,10<br>1-8,11-22 |
| X<br>A | NEWBERG, A. B. et al., Safety, biodistribution, and dosimetry of 123I-IMPY:A novel amyloid plaque-imaging agent for the diagnosis of Alzheimer's disease, The Journal of Nuclear Medicine, 2006.05, Vol.47, No.5, p.748-754 | 5-8,19-22<br>1-4,9-18 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007002540 A
- JP 2004506723 T
- JP 2005504055 T
- JP 2005512945 T
- JP 2002523383 T
- WO 2007063946 A
- WO O3106439 A
- WO 03106439 A


### Non-patent literature cited in the description


- **J. A. Hardy ; G. A. Higgins.** Alzheimer's Disease: The Amyloid Cascade Hypothesis. *Science,* 1992, vol. 256, 184-185
- **G. McKhann et al.** Clinical diagnosis of Alzheimer's disease: Report of the NINCDS-ADRDA Work Group under the auspices of Department of Health and Human Services Task Force on Alzheimer's Disease. *Neurology,* 1984, vol. 34, 939-944
- **Z.-P. Zhuang et al.** Radioiodinated Styrylbenzenes and Thioflavins as Probes for Amyloid Aggregates. *J. Med. Chem.,* 2001, vol. 44, 1905-1914
- **Masahiro Ono et al.** 11C-laheled stilbene derivatives as Aβ-aggregate-specific PET imaging agents for Alzheimer's disease. *NuclearMedicine and Biology,* 2003, vol. 30, 565-571
- **H. F. Kung et al.** Novel Stilbenes as Probes for amyloid plaques. *J. American Chemical Society,* 2001, vol. 123, 12740-12741
- **Zhi-Ping Zhuang et al.** IBOX (2- (4'-dimethylaminophenyl)-6- iodobensoxazole): a ligand for imaging amyloid plaques in the brain. *Nuclear Medicine and Biology,* 2001, vol. 28, 887-894
- **Furumoto Y et al.** [11C]BF-227: A New C-Labeled 2-Ethenylbenzoxazole Derivative for Amyloid-β Plaques Imaging. *European Journal of Nuclear Medicine and Molecular Imagine,* 2005, vol. 32 (1), 759
- **Eric D. Agdeppa et al.** 2-Dialkylamino-6-Acylmalononitrile Substituted Naphthalenes (DDNP Analogs): Novel Diagnostic and Therapeutic Tools in Alzheimer's Disease. *Molecular Imaging and Biology,* 2003, vol. 5, 404-417
- **Zhi-Ping Zhuang et al.** Structure-Activity Relationship of Imidazo[1,2-a]pyridines as Ligands for Detecting β-Amnyloid Plaques in the Brain. *J. Med. Chem,* 2003, vol. 46, 237-243
- **W. E. Klunk et al.** Imaging brain amyloid in Alzheimer's disease with Pittsburgh Compound-B. *Ann. Neurol.,* 2004, vol. 55, 306-319
- **Nicolaas P. L. G. Verhoeff et al.** In-Vivo Imaging of Alzheimer Disease β-Amyloid With [11C] SB-13 PET. *American Journal of Geriatric Psychiatry,* 2004, vol. 12, 584-595
- **Hiroyuki Arai et al.** [11C]-BF-227 AND PET to visualize Amyloid in Alzheimer's Disease Patients. *Alzheimer's & Dementia: The Journal of the Alzheimer's Association,* 2006, vol. 2 (1), 312
- **Christopher M. Clark et al.** Imaging Amyloid with I123 IMPY SPECT. *Alzheimer's & Dementia: The Journal of the Alzheimer's Association,* 2006, vol. 2 (1), 342
- **Zhi-Ping Zhuang et al.** *Nuclear Medicine and Biology,* 2001, vol. 28, 887-894
- **H. F. Kung et al.** *J. Am. Chem. Soc.,* 2001, vol. 123, 12740-12741
- **Masahiro Ono et al.** *Nuclear Medicine and Biology,* 2003, vol. 30, 565-571
- **Joseph G. Lombardino.** *Journal of Medicinal Chemistry,* 1981, vol. 24, 39-42
- **King, L. Carroll ; Ostrum, G. Kenneth.** *Journal of Organic Chemistry,* 1964, vol. 29 (12), 3459-3461
- **Zhi-Ping Zhuang et al.** *J. Med. Chem.,* 2003, vol. 46, 237-243
- **Naiki, H. et al.** *Laboratory Investigation,* 1996, vol. 74, 374-383
- **Douglas D. Dischino et al.** *J. Nucl. Med.,* 1983, vol. 24, 1030-1033